# EUROPÄISCHE PATENTSCHRIFT

(11) **EP 2 554 183 B1**
(45) Veröffentlichungstag und Bekanntmachung des Hinweises auf die Patenterteilung: **04.04.2018**
(21) Anmeldenummer: 12190525.1
(22) Anmeldetag: 11.11.2010
(51) Int. Cl.: A61K 38/28, A61K 38/26, A61P 3/10, A61K 47/10, A61K 47/18, A61K 9/00

(54) **Pharmazeutische Zusammensetzung umfassend einen GLP-1-Agonisten, ein Insulin und Methionin**
Pharmaceutical composition comprising a GLP-1-agonist, an insulin, and methionine
Composition pharmaceutique comprenant un agoniste GLP-1, de l'insuline et de la méthionine

(30) Priorität: 13.11.2009 DE 102009052831; 18.05.2010 DE 102010020902
(43) Veröffentlichungstag der Anmeldung: 06.02.2013
(62) Teilanmeldung aus: 10776998.6
(73) Patentinhaber: Sanofi-Aventis Deutschland GmbH, 65929 Frankfurt am Main (DE)
(72) Erfinder: Hagendorf, Annika, 65926 Frankfurt am Main (DE); Hauck, Gerrit, 65926 Frankfurt am Main (DE); Müller, Werner, 65926 Frankfurt am Main (DE); Schoettle, Isabell, 65926 Frankfurt am Main (DE); Siefke-Henzler, Verena, 65926 Frankfurt am Main (DE); Tertsch, Katrin, 65926 Frankfurt am Main (DE)
(74) Vertreter: Weickmann & Weickmann PartmbB

(56) Entgegenhaltungen:
- WO-A1-03/066084
- WO-A1-2007/104786
- WO-A2-2005/028516
- WO-A2-2008/133908
- TEWS D ET AL: "Enhanced protection against cytokine- and fatty acid-induced apoptosis in pancreatic beta cells by combined treatment with glucagon-like peptide-1 receptor agonists and insulin analogues", HORMONE AND METABOLIC RESEARCH, THIEME-STRATTON, STUTTGART, DE, Bd. 40, Nr. 3, 1. März 2008 (2008-03-01), Seiten 172-180, XP009130880, ISSN: 0018-5043, DOI: DOI:10.1055/S-2008-1042426
- ARNOLDS SABINE ET AL: "Insulin Glargine (GLAR) plus Metformin (MET): An Efficacious and Safe Regimen That Can Be Combined with Exenatide (EXE) or Sitagliptin (SITA)", DIABETES, AMERICAN DIABETES ASSOCIATION, US, Bd. 58, Nr. Suppl. 1, 1. Juni 2009 (2009-06-01), Seite A141, XP009130958, ISSN: 0012-1797

## Beschreibung

Gegenstand der vorliegenden Anmeldung ist eine flüssige Zusammensetzung gemäß Anspruch 1 umfassend einen GLP-1-Agonisten oder/und ein pharmakologisch tolerierbares Salz davon, ein Insulin oder/und ein pharmakologisch tolerierbares Salz davon und gegebenenfalls mindestens einen pharmazeutisch akzeptablen Hilfsstoff, wobei die Zusammensetzung dadurch gekennzeichnet ist, dass sie Methionin enthält.
Ein weiterer Gegenstand ist die erfindungsgemäße Zusammensetzung zur Behandlung von Diabetes mellitus. Noch ein weiterer Gegenstand ist die Verwendung einer erfindungsgemäßen Zusammensetzung zur Herstellung eines Arzneimittel zur Behandlung von Diabetes mellitus. Außerdem wird ein Verfahren zur Herstellung einer erfindungsgemäßen Zusammensetzung beschrieben, umfassend Formulieren eines GLP-1-Agonisten oder/und eines pharmakologisch tolerierbares Salzes davon mit einem Insulin oder/und einem pharmazeutisch akzeptablen Salz davon, Methionin und gegebenenfalls mindestens einem pharmazeutisch akzeptablen Hilfsstoff. Übliche Zusammensetzungen von Insulin und GLP-1-Verbindungen enthalten ein Isotonisierungsmittel, einen Puffer zur Einstellung des pH-Wertes und einen Konservierungsstoff. Ein weiterer häufig verwendeter Bestandteil von Insulinzusammensetzungen ist Zink, welches mit Insulin einen Komplex bildet. Hierdurch läßt sich eine verzögerte Wirkung des Insulins erzielen.

WO 2004/035623 (Zealand Pharmaceuticals) offenbart eine flüssige Zusammensetzung umfassend ein stabilisiertes Exendin, 50 mM Histidin, 100 bis 200 mM Saccharose, Mannitol oder einen anderen akzeptablen Zucker, 20 mM Methionin, 20 mM Asparagin-Glutamin oder Asp mit einem pH von 5,3. Die Stabilisierung wird durch bestimmte Modifikationen der Aminosäurebausteine von Exendin-4(1-39) bewirkt, z.B. an den Positionen Gln13, Met14, Trp25 oder Asn28. Diese Zusammensetzung enthält kein Insulin.
WO 2005/046716 (Novo Nordisk) offenbart flüssige Zusammensetzungen, welche Liraglutid und Insulin Aspart, einen Puffer pH 7,7, Poloxamer 188 als oberflächenaktive Substanz, Phenol, Propylenglycol und ggf. Zink enthält. Ohne Poloxamer 188 waren die Zusammensetzungen instabil. Durch Polysorbat 20 konnte eine Stabilisierung erzielt werden.
WO 2006/029634 (Novo Nordisk) betrifft flüssige pharmazeutische Zusammensetzungen, welche ein insulinotropes Peptid (GLP-1-Agonist), ein Insulinpeptid und einen Liganden für His^{B10} (Ligand von His an Position 10 der B-Kette von Insulin) enthalten. Die Zusammensetzung kann als oberflächenaktive Substanz Polysorbat-20 oder Poloxamer 188 enthalten. Spezifische in diesem Dokument offenbarte Zusammensetzungen enthalten humanes Insulin oder humanes B28-Asp-Insulin (Insulin Aspart), Liraglutid (GLP-1-Agonist), Glycerin als Isotonisierungsmittel, Zinkacetat, pH 7,4 oder 7,9. Je nach Menge des eingesetzten Insulins oder von Liraglutid wurden diese Zusammensetzungen teilweise schon nach 15 Tagen Lagerung bei Raumtemperatur instabil. Stabilität dieser Zusammensetzungen wurde durch Zugabe eines Liganden für His^{B10} erzielt. Weitere Formulierungen bestanden aus Liraglutid, Insulin Aspart oder Detemir, Propylenglycol, Phenol und Phosphatpuffer pH 7,7. Diese Zusammensetzungen waren praktisch sofort instabil. Zugabe von Poloxamer-188 oder Polysorbat-20 und eines Liganden für His^{B10} führte zu einer Stabilisierung.

WO 2006/051103 (Novo Nordisk) offenbart flüssige Zusammensetzungen, welche Detemir (ein Basalinsulin), Liraglutid (GLP-1-Verbindung) und als oberflächenaktive Substanz Poloxamer 188 oder Polysorbat 20 enthalten. Weitere Bestandteile sind Phenol, NaCl, Propylenglycol, Zinkacetat und Natriumphosphat- oder Glycylglycinpuffer (pH 7,7) enthalten. m-Kresol ist in einigen dieser Zusammensetzungen enthalten. Durch die Zugabe von Poloxamer 188 oder Polysorbat 20 konnten die Zusammensetzungen stabilisiert werden.

WO 2008/124522 (Biodel) betrifft Zusammensetzungen, welche ein Insulin, einen Zinkchelator (z.B. EDTA oder EGTA), und ein GLP-1-Analogon enthalten.

Weltweit leiden etwa 120 Mio. Menschen an Diabetes mellitus. Darunter sind etwa 12 Mio. Typ I-Diabetiker, für die die Substitution der fehlenden endokrinen Insulinsekretion die einzige derzeit mögliche Therapie darstellt. Die Betroffenen sind lebenslang, in der Regel mehrmals täglich, auf Insulininjektionen angewiesen. Im Gegensatz zum Typ I-Diabetes besteht beim Typ II-Diabetes nicht grundsätzlich ein Mangel an Insulin, jedoch wird in einer Vielzahl von Fällen, vor allem im fortgeschrittenen Stadium, die Behandlung mit Insulin, gegebenenfalls in Kombination mit einem oralen Antidiabetikum, als günstigste Therapieform angesehen.

Beim Gesunden ist die Insulinfreisetzung durch den Pankreas strikt an die Konzentration der Blutglucose gekoppelt. Erhöhte Blutglucosespiegel, wie sie nach Mahlzeiten auftreten, werden durch eine entsprechende Steigerung der Insulinsekretion rasch kompensiert. Im nüchternen Zustand sinkt der Plasmainsulinspiegel auf einen basalen Wert ab, der ausreicht, eine kontinuierliche Versorgung insulinsensitiver Organe und Gewebe mit Glucose zu gewährleisten und die hepatische Glucoseproduktion in der Nacht niedrig zu halten. Der Ersatz der körpereigenen Insulinsekretion durch exogene, meist subkutane Applikation von Insulin erreicht in der Regel die oben beschriebene Qualität der physiologischen Regulation der Blutglucose nicht annähernd. Häufig kommt es zu Entgleisungen der Blutglucose nach oben oder unten, die in ihren schwersten Formen lebensbedrohlich sein können. Daneben stellen jedoch auch über Jahre erhöhte Blutglucosespiegel ohne anfängliche Symptome ein erhebliches Gesundheitsrisiko dar. Die großangelegte DCCT-Studie in den USA (The Diabetes Control and Complications Trial Research Group (1993) N. Engl. J. Med. 329, 977-986) wies eindeutig nach, dass chronisch erhöhte Blutglucosespiegel wesentlich für die Entwicklung diabetischer Spätschäden verantwortlich sind. Diabetische Spätschäden sind mikro- und makrovaskuläre Schädigungen, die sich u.U. als Retino-, Nephro-, oder Neuropathie manifestieren und zu Erblindung, Nierenversagen sowie dem Verlust von Extremitäten führen und darüber hinaus mit einem erhöhten Risiko für Herz/Kreislauferkrankungen einhergehen. Daraus ist abzuleiten, dass eine verbesserte Therapie des Diabetes in erster Linie darauf abzielen muss, die Blutglucose möglichst eng im physiologischen Bereich zu halten. Nach dem Konzept der intensivierten Insulintherapie soll dies durch mehrmals tägliche Injektionen von schnell und langsam wirkenden Insulinzubereitungen erreicht werden. Rasch wirkende Formulierungen werden zu den Mahlzeiten gegeben, um den postprandialen Anstieg der Blutglucose auszugleichen. Langsam wirkende Basalinsuline sollen die Grundversorgung mit Insulin insbesondere während der Nacht sicherstellen, ohne zu einer Hypoglykämie zu führen.

Insulin ist ein Polypeptid aus 51 Aminosäuren, die sich auf 2 Aminosäureketten verteilen: die A Kette mit 21 Aminosäuren und die B-Kette mit 30 Aminosäuren. Die Ketten sind durch 2 Disulfidbrücken miteinander verbunden. Insulinzubereitungen werden seit vielen Jahren zur Diabetestherapie eingesetzt. Dabei werden nicht nur natürlich vorkommende Insuline verwendet, sondern neuerdings auch Insulinderivate und -analoga.

Insulinanaloga sind Analoga von natürlich vorkommenden Insulinen, nämlich Humaninsulin oder tierischen Insulinen, welche sich durch Substitution wenigstens eines natürlich auftretenden Aminosäurerestes mit anderen Aminosäuren und/oder Addition/Entfernen wenigstens eines Aminosäurerestes von dem entsprechenden, ansonsten gleichen natürlich vorkommenden Insulin unterscheiden. Es kann sich dabei auch um Aminosäuren handeln, die nicht natürlich vorkommen.

Insulinderivate sind Derivate von natürlich vorkommendem Insulin oder einem Insulinanalogon, welche durch chemische Modifizierung erhalten werden. Die chemische Modifikation kann z.B. in der Addition einer oder mehrerer bestimmter chemischer Gruppen an eine oder mehrere Aminosäuren bestehen. In der Regel haben Insulinderivate und Insulinanaloga gegenüber humanem Insulin eine etwas veränderte Wirkung.

Insulinanaloga mit beschleunigtem Wirkungseintritt werden in EP 0 214 826, EP 0 375 437 und EP 0 678 522 beschrieben. EP 0 214 826 bezieht sich u.a. auf Substitutionen von B27 und B28. EP 0 678 522 beschreibt Insulinanaloga, die in der Position B29 verschiedene Aminosäuren, vorzugsweise Prolin, aufweisen, jedoch nicht Glutaminsäure. EP 0 375 437 umfaßt Insulinanaloga mit Lysin oder Arginin in B28, die optional zusätzlich in B3 und/oder A21 modifiziert sein können.

In der EP 0 419 504 werden Insulinanaloga offenbart, die gegen chemische Modifikationen geschützt sind, indem Asparagin in B3 und wenigstens eine weitere Aminosäure in den Positionen A5, A15, A18 oder A21 verändert sind.

In der WO 92/00321 werden Insulinanaloga beschrieben, bei denen wenigstens eine Aminosäure der Positionen B1-B6 durch Lysin oder Arginin ersetzt ist. Derartige Insuline weisen gemäß WO 92/00321 eine verlängerte Wirkung auf. Eine verzögerte Wirkung weisen auch die in der EP-A 0 368 187 beschriebenen Insulinanaloga auf.

Die auf dem Markt befindlichen Insulinzubereitungen von natürlich vorkommenden Insulinen zur Insulinsubstitution unterscheiden sich in der Herkunft des Insulins (z.B. Rind, Schwein, Humaninsulin), sowie der Zusammensetzung, womit das Wirkprofil (Wirkeintritt und Wirkdauer) beeinflusst werden kann. Durch Kombination verschiedener Insulinpräparate lassen sich unterschiedlichste Wirkprofile erzielen und möglichst physiologische Blutzuckerwerte einstellen. Die rekombinante DNA-Technologie ermöglicht heutzutage die Herstellung von solchen modifizierten Insulinen. Hierzu zählt Insulin Glargin (Gly(A21)-Arg(B31)-Arg(B32)-Humaninsulin, Lantus) mit einer verlängerten Wirkdauer. Insulin Glargin wird als saure, klare Lösung injiziert und fällt aufgrund seiner Lösungseigenschaften im physiologischen pH-Bereich des Subkutangewebes als stabiles Hexamerassoziat aus. Insulin Glargin wird einmal täglich injiziert und zeichnet sich gegenüber anderen langwirksamen Insulinen durch sein flaches Serumprofil und die damit verbundene Reduktion der Gefahr nächtlicher Hypoglykämien aus (Schubert-Zsilavecz et al., 2:125-130(2001)).

Die spezifische Zubereitung des Insulin Glargins, die zur verlängerten Wirkdauer führt, ist durch einen klare Lösung mit saurem pH-Wert gekennzeichnet.

Exendine sind eine Gruppe von Peptiden, welche die Blutglucosekonzentrationen senken können. Exendine weisen eine gewisse Ähnlichkeit der Sequenz zu GLP-1(7-36) auf (53%, Goke et al. J. Biol Chem 268, 19650-55). Exendin-3 und Exendin-4 stimulieren einen Anstieg der zellulären cAMP-Produktion in Azinuszellen des Meerschweinchenpankreas durch Interaktion mit Exendinrezeptoren (Raufman, 1996, Reg. Peptides 61:1-18). Exendin-3 bewirkt im Gegensatz zu Exendin-4 einen Anstieg der Amylasefreisetzung in Azinuszellen des Pankreas. Exendine wirken als GLP-1-Agonisten.

Glucagon-like peptide 1 (GLP-1) ist ein endokrines Hormon, welches die Insulinantwort nach oraler Aufnahme von Glucose oder Fett erhöht. GLP-1 senkt generell die Glucagonkonzentrationen, verlangsamt die Magenentleerung, stimuliert die (Pro-)Insulin-Biosynthese, erhöht die Empfindlichkeit gegenüber Insulin und stimuliert die Insulin-unabhängige Glycogen-Biosynthese (Holst (1999), Curr. Med. Chem 6:1005, Nauck et al. (1997) Exp Clin Endocrinol Diabetes 105: 187, Lopez-Delgado et al. (1998) Endocrinology 139:2811). Humanes GLP-1 weist 37 Aminosäurereste auf (Heinrich et al., Endocrinol. 115:2176 (1984), Uttenthal et al., J Clin Endocrinol Metabol (1985) 61:472). Aktive Fragmente von GLP-1 schließen GLP-1 (7-36) und GLP-1(7-37) ein.

Exendin-3, Exendin-4 und Exendinagonisten wurden für die Behandlung von Diabetes mellitus und die Prävention von Hyperglykämie vorgeschlagen, wobei sie die Magenmotilität und -entleerung vermindern (US 5,424,286 und WO98/05351). Exendin-Analoga können gekennzeichnet sein durch Aminosäuresubstitutionen und/oder C-terminalen Trunkierung der nativen Exendin-4-Sequenz. Derartige Exendin-Analoga sind beschrieben in WO 99/07404, WO 99/25727 und WO 99/25728.

Die Festphasensynthese von AVE0010 ist in WO 01/04156 A1 beschrieben. AVE0010 weist die Sequenz: desPro³⁶Exendin-4(1-39)-Lys₆-NH₂ auf. Diese Substanz ist als SEQ ID NO:93 in WO 01/04156 veröffentlicht:

H-G-E-G-T-F-T-S-D-L-S-K-Q-M-E-E-E-A-V-R-L-F-I-E-W-L-K-N-G-G-P-S-S-G-A-P-P-S-
K-K-K-K-K-K-NH2          (SEQ ID NO:1)

Exendin-4 (39 AS) weist die Sequenz auf:

H-G-E-G-T-F-T-S-D-L-S-K-Q-M-E-E-E-A-V-R-L-F-I-E-W-L-K-N-G-G-P-S-S-G-A-P-P-P-
S-NH2 (SEQ ID NO:2)

Exendin-3 weist die Sequenz auf (J. Bio. Chem., 267, 1992, 7402-7405):

H-His-Ser-Asp-Gly-Thr-Phe-Thr-Ser-Asp-Leu-Ser-Lys-Gln-Met-Glu-Glu-Glu-Ala-Val-
Arg-Leu-Phe-Ile-Glu-Trp-Leu-Lys-Asn-Gly-Gly-Pro-Ser-Ser-Gly-Ala-Pro-Pro-Pro-Ser-
NH2 (SEQ ID NO: 3)

GLP-1 weist die Sequenz auf:
H-A-E-G-T-F-T-S-D-V-S-S-Y-L-E-G-Q-A-A-K-E-F-I-A-W-L-V-K-G-R-NH₂ (SEQ ID NO: 4)

Die Aufgabe der vorliegenden Erfindung bestand darin, die Stabilität von flüssigen Formulierungen umfassend einen GLP-1-Agonisten und ein Insulin zu erhöhen.

Insbesondere soll die physikalische und chemische Integrität verbessert werden. Diese Aufgabe wurde dadurch gelöst, dass der GLP-1-Agonist und das Insulin mit Methionin formuliert wurden.
Es wurde gefunden, dass Methionin die Lagerstabilität einer Zusammensetzung umfassend einen GLP-1-Agonisten wie AVE0010 und eines Insulins wie Insulin Glargin erhöhen kann. Methionin beeinflusst die physikalische Integrität dieser Zusammensetzungen nicht.
Die Stabilität von pharmazeutisch wirksamen Polypeptiden kann durch verschiedene Mechanismen beeinträchtigt werden. Hierzu gehört der pH, die Temperatur, Licht und die Wirkungen bestimmter Bestandteile.
In Zusammenhang mit der vorliegenden Erfindung wurde gefunden, dass eine Reihe üblicher Bestandteile von Insulinformulierungen oder von Formulierungen von GLP-1-Agonisten nachteilig für die chemische oder/und physikalische Integrität und die Lagerstabilität von Formulierungen sind, welche ein Insulin und einen GLP-1-Agonisten enthalten. Dies sind zum Beispiel Acetat, Polysorbat 20, Polysorbat 80, Poloxamer 188, Benzalkoniumchlorid und Lysin. Die erfindungsgemäßen Zusammensetzungen sind daher bevorzugt frei von diesen Bestandteilen.

Ein Gegenstand der vorliegenden Erfindung ist eine flüssige gemäß Anspruch 1 umfassend einen GLP-1-Agonisten oder/und ein pharmakologisch tolerierbares Salz davon, ein Insulin oder/und ein pharmakologisch tolerierbares Salz davon und gegebenenfalls mindestens einen pharmazeutisch akzeptablen Hilfsstoff, wobei die Zusammensetzung dadurch gekennzeichnet ist, dass sie Methionin enthält.

Die erfindungsgemäße Zusammensetzung enthält Methionin in einer Menge von 0,5 mg/mL bis 20 mg/mL, stärker bevorzugt in einer Menge von 1 mg/mL bis 5 mg/mL, besonders bevorzugt in einer Menge von 3,0 mg/mL. Methionin kann in der D-Form eingesetzt werden. Ebenso kann Methionin in der L-Form eingesetzt werden. Ebenso können Gemische der D- und der L-Form in beliebigen Proportionen eingesetzt werden.

Insbesondere ist die erfindungsgemäße Zusammensetzung frei von Tensiden, wie Polyolen und Partial- und Fettsäureester und -ether mehrwertiger Alkohole wie des Glycerins und Sorbitols. Die erfindungsgemäßen Zusammensetzungen sind insbesondere frei von Partial- und Fettsäureester und -ether des Glycerins und Sorbitols ausgewählt aus einer Gruppe umfassend Span®, Tween®, Myrj®, Brij®, Cremophor®. Ferner sind die erfindungsgemäßen Zusammensetzung insbesondere frei von Polyolen ausgewählt werden aus der Gruppe Polypropylenglycole, Polyethylenglycole, Poloxamere, Pluronics, Tetronics. Insbesondere ist die erfindungsgemäße Zusammensetzung frei von mindestens einer Substanz ausgewählt aus Polysorbat, Polysorbat und Poloxamer.

Insbesondere ist die erfindungsgemäße Zusammensetzung im wesentlichen frei, bevorzugt frei von Polysorbat, wie z. B. Polysorbat 20.

Insbesondere ist die erfindungsgemäße Zusammensetzung im wesentlichen frei, bevorzugt frei von Polysorbat 80.

Insbesondere ist die erfindungsgemäße Zusammensetzung im wesentlichen frei, bevorzugt frei von Poloxamer, wie z. B. Poloxamer 188.

Insbesondere ist die erfindungsgemäße Zusammensetzung im wesentlichen frei, bevorzugt frei von Benzalkoniumchlorid.

Insbesondere ist die erfindungsgemäße Zusammensetzung im wesentlichen frei, bevorzugt frei von Histidin.

Insbesondere ist die erfindungsgemäße Zusammensetzung im wesentlichen frei, bevorzugt frei von EDTA, insbesondere Natrium-EDTA.

Insbesondere ist die erfindungsgemäße Zusammensetzung im wesentlichen frei, bevorzugt frei von Histidin und Natrium-EDTA.

Die erfindungsgemäße Zusammensetzung kann eine oder mehrere Substanzen enthalten, welche üblicherweise zur Pufferung des pH-Wertes verwendet werden (Puffersubstanzen). Beispiele derartiger Puffersubstanzen sind Acetat, Citrat und Phosphat. Insbesondere kann die erfindungsgemäße Zusammensetzung eine oder mehrere Substanzen, welche üblicherweise zur Pufferung des pH-Wertes verwendet werden, in einer Menge enthalten, welche zum Beispiel als Gegenion für den GLP-1-Agonisten oder/und das Insulin ausreichend ist. Die erfindungsgemäße Zusammensetzung kann eine oder mehrere Puffersubstanzen zum Beispiel jeweils in einer Menge von bis zu 1 mg/ml, bis zu 0,5 mg/ml, bis zu 0,1 mg/ml, bis zu 0,05 mg/ml, bis zu 0,02 mg/ml oder bis zu 0,01 mg/ml enthalten. Die erfindungsgemäße Zusammensetzung kann ebenso im wesentlichen frei von Puffersubstanzen sein. Bevorzugt ist die erfindungsgemäße Zusammensetzung frei von Puffersubstanzen.

Die erfindungsgemäße Zusammensetzung kann Acetat enthalten, zum Beispiel in einer Menge von bis zu 1 mg/ml, bis zu 0,5 mg/ml, bis zu 0,1 mg/ml, bis zu 0,05 mg/ml, bis zu 0,02 mg/ml oder bis zu 0,01 mg/ml. Diese Mengen sind zum Beispiel als Gegenion für den GLP-1-Agonisten ausreichend. Ebenso kann die erfindungsgemäße Zusammensetzung im wesentlichen frei von Acetat sein. Bevorzugt ist die erfindungsgemäße Zusammensetzung frei von Acetat.

Die erfindungsgemäße Zusammensetzung kann Citrat enthalten, zum Beispiel in einer Menge von bis zu 1 mg/ml, bis zu 0,5 mg/ml, bis zu 0,1 mg/ml, bis zu 0,05 mg/ml, bis zu 0,02 mg/ml oder bis zu 0,01 mg/ml. Diese Mengen sind zum Beispiel als Gegenion für den GLP-1-Agonisten ausreichend. Ebenso kann die erfindungsgemäße Zusammensetzung im wesentlichen frei von Citrat sein. Bevorzugt ist die erfindungsgemäße Zusammensetzung frei von Citrat.

Die erfindungsgemäße Zusammensetzung kann Phosphat enthalten, zum Beispiel in einer Menge von bis zu 1 mg/ml, bis zu 0,5 mg/ml, bis zu 0,1 mg/ml, bis zu 0,05 mg/ml, bis zu 0,02 mg/ml oder bis zu 0,01 mg/ml. Diese Mengen sind zum Beispiel als Gegenion für den GLP-1-Agonisten ausreichend. Ebenso kann die erfindungsgemäße Zusammensetzung im wesentlichen frei von Phosphat sein. Bevorzugt ist die erfindungsgemäße Zusammensetzung frei von Phosphat.

Die pharmazeutische Zusammensetzung der vorliegenden Erfindung kann einen saurem oder physiologischen pH aufweisen. Ein saurer pH Bereich liegt vorzugsweise im Bereich von pH 1 - 6,8, pH 3,5 - 6,8, oder pH 3,5 - 5. Ein physiologischem pH liegt bevorzugt im Bereich von pH 2,5 -8,5, stärker bevorzugt pH 4,0 bis 8,5, noch stärker bevorzugt pH 6,0 bis 8,5. Besonders bevorzugt ist ein pH-Wert von etwa 4,5. Zur Einstellung des pH-Wertes sind physiologisch unbedenkliche verdünnte Säuren (typischerweise HCl) bzw. Laugen (typischerweise NaOH) geeignet.

Die erfindungsgemäße Zusammensetzung kann ein geeignetes Konservierungsmittel enthalten. Geeignete Konservierungsmittel sind z.B. Phenol, m-Kresol, Benzylalkohol und/oder p-Hydroxybenzoesäureester. Bevorzugt ist m-Kresol. Es kann aber auch auf Konservierungsmittel verzichtet werden.

Die erfindungsgemäße Zusammensetzung kann Zinkionen enthalten. Die Konzentration der Zinkionen liegt vorzugsweise im Bereich von 1 µg/ml bis 2 mg/ml, stärker bevorzugt von 5 µg bis 200 µg Zink/ml, insbesondere bei max. 0,06 mg/ml, besonders bevorzugt bei 0,06 mg/ml.

Ferner kann die erfindungsgemäße Zusammensetzung geeignete Isotonisierungsmittel enthalten. Geeignet sind z.B. Glycerin, Dextrose, Lactose, Sorbitol, Mannitol, Glucose, NaCl, Calcium- oder Magnesium-Verbindungen wie CaCl₂ etc. Die Konzentrationen von Glycerin, Dextrose, Lactose, Sorbitol, Mannitol und Glucose liegen üblicherweise im Bereich von 100 - 250 mM, NaCl in einer Konzentration von bis zu 150 mM. Bevorzugt ist Glycerin. Insbesondere ist bevorzugt 85 %iges Glycerin bei 20,0 mg/ml.

Ferner kann die erfindungsgemäße Zusammensetzung weitere Zusätze wie z.B. Salze enthalten, welche die Freigabe des wenigstens einen Insulins retardieren. Bevorzugt ist die Zusammensetzung frei von diesen Zusätzen.

Insbesondere ist die Zusammensetzung zur parenteralen Verabreichung vorgesehen. Die erfindungsgemäße Zusammensetzung ist vorzugsweise eine injizierbare Zusammensetzung, stärker bevorzugt zur subkutanen Injektion. Insbesondere ist die Zusammensetzung der vorliegenden Erfindung zur Injektion einmal täglich geeignet.

Insbesondere weist die erfindungsgemäße Formulierung nach einer Lagerung von 1 Monat, 2 Monaten, 4 Monaten oder 6 Monaten bei einer Temperatur von +5°C oder 25°C eine Aktivität von wenigstens 80 %, wenigstens 90 %, wenigstens 95 %, oder wenigstens 98% der Aktivität zum Ausgangszeitpunkt auf.

"Aktivität" kann in der vorliegenden Anmeldung die Aktivität des Insulins bedeuten, welches in der erfindungsgemäßen Formulierung eingesetzt wird. Verfahren zur Bestimmung der Aktivität von Insulin sind dem Fachmann bekannt.

"Aktivität" kann in der vorliegenden Anmeldung ebenso die Aktivität des GLP-1-Agonisten bedeuten, welcher in der erfindungsgemäßen Formulierung eingesetzt wird. Verfahren zur Bestimmung der Aktivität eines GLP-1-Agonisten sind dem Fachmann bekannt.

Die erfindungsgemäße Formulierung weist insbesondere nach Lagerung von 1 Monat, 2 Monaten, 4 Monaten oder 6 Monaten eine chemische Integrität auf. Chemische Integrität bedeutet insbesondere, dass sie nach einer Lagerung bei einer Temperatur von +5°C, 25°C oder 40°C wenigstens 80 %, wenigstens 90 %, wenigstens 95 %, oder wenigstens 98% des Wirkstoffs zum Ausgangszeitpunkt in im wesentlichen chemisch unveränderter Form enthält.

Chemische Integrität kann die chemische Integrität des GLP-1-Agonisten bedeuten. GLP-1-Agonisten können einen Methioninrest enthalten (z.B. Position 14 in AVE0010). Chemische Integrität des GLP-1-Agonisten bedeutet insbesondere, dass die Oxidation dieses Methioninrestes verhindert wird.

Chemische Integrität kann ebenso die chemische Integrität des Insulins bedeuten. Bevorzugt bedeutet chemische Integrität die Integrität des Insulins und des GLP-1-Agonisten.
Die erfindungsgemäße Formulierung weist insbesondere nach Lagerung von 1 Monat, 2 Monaten, 4 Monaten oder 6 Monaten eine physikalische Integrität auf. Physikalische Integrität bedeutet insbesondere, dass sie nach einer Lagerung bei einer Temperatur von +5°C, 25°C oder 40°C wenigstens 80 %, wenigstens 90 %, wenigstens 95 %, oder wenigstens 98% des Wirkstoffs zum Ausgangszeitpunkt in im wesentlichen physikalisch unveränderter Form enthält.
Physikalische Integrität kann die Integrität des GLP-1-Agonisten bedeuten. Ebenso kann die physikalische Integrität die Integrität des Insulins bedeuten. Physikalische Integrität bedeutet insbesondere, dass der GLP-1-Agonist oder/und das Insulin keine Aggregationen bilden, wie z.B. Fibrillen.
Bevorzugt bedeutet physikalische Integrität die Integrität des Insulins und des GLP-1-Agonisten.
Der GLP-1-Agonist ist bevorzugt ausgewählt aus der Gruppe bestehend aus Exendin-3 und Analoga und Derivaten davon, Exendin-4 und Analoga und Derivaten davon, und wobei der GLP-1-Agonist stärker bevorzugt ausgewählt ist aus der Gruppe bestehend aus AVE0010 und Exendin-4.
Exendin-3, Analoga und Derivate von Exendin-3, Exendin-4 und Analoga und Derivate von Exendin-4 können in WO 01/04156, WO 98/30231, US 5,424,286, in der EP-Anmeldung 99 610043.4 und in WO 2004/005342 gefunden werden. Das in diesen Dokumenten beschriebene Exendin-3, Exendin-4 und die dort beschriebenen Analoga und Derivate davon können mittels der hierin beschriebenen Verfahren synthetisiert werden, wobei ggf. nach Abschluss des Verfahrens Modifikationen durchgeführt werden.

Die Sequenzen von AVE0010 (SEQ ID NO:1), Exendin-4 (SEQ ID NO:2) und Exendin-3 (SEQ ID NO:3) zeigen einen hohen Grad an Übereinstimmung. Die Sequenzen von AVE0010 und Exendin-4 sind in den Positionen 1-37 identisch. Die Sequenz 1-39 aus Exendin-4 ist an 37 der 39 Positionen (94%) identisch mit der Exendin-3-Sequenz an den Positionen 48-86. Anhand der Sequenzen kann der Fachmann hierin angegebene Positionsangaben, welche sich auf eine bestimmte Sequenz beziehen (z.B. auf die Sequenz von AVE0010 oder Exendin-4), ohne weiteres auf andere Sequenzen umrechnen.
Analoga und Derivate von Exendin-3 oder/und Exendin-4 enthalten insbesondere eine modifizierte Aminosäuresequenz. Es können zum Beispiel einzelne Aminosäuren deletiert sein (z.B. desPro36, desPro37, desAsp28, desMet(O)14 in Exendin-4 und den entsprechenden Positionen in Exendin-3). Ebenso können einzelne Positionen ersetzt werden (z. B. Met(O)¹⁴, Trp(O₂)²⁵, IsoAsp²⁸, Asp²⁸ Pro³⁸ in Exendin-4 und den entsprechenden Positionen in Exendin-3), wobei auch nicht-natürliche Aminosäuren wie Met(O) (Methioninsulfioxid oder Methioninsulfon), Trp(O₂) (N-Formylkynurenin) oder/und IsoAsp (β-Aspartat oder Isoaspartat) eingesetzt werden können. Nicht-natürliche Aminosäuren können ohne weiteres in Form entsprechender Aminosäurebausteine in die Sequenz eingeführt werden.

Ferner können der C-Terminus oder/und der N-Terminus modifiziert sein, z.B. durch eine zusätzliche Sequenz wie -(Lys)-, -(Lys)₂-, -(Lys)₃-, -(Lys)₄-, -(Lys)₅-, -(Lys)₆-, -Asn-(Glu)₅-, wobei -(Lys)₄-, -(Lys)₅-, -(Lys)₆- , -Asn-(Glu)₅- bevorzugt sind. Die Carboxylgruppe am C-Terminus ist bevorzugt als Säureamidgruppe (-NH₂) modifiziert. Ggf. wird die Modifikation am C-Terminus oder/und am N-Terminus als weiterer Verfahrensschritt nach Abschluss der Synthese durchgeführt.

Pharmazeutisch tolerierbare Salze können in einem weiteren Verfahrensschritt nach Abschluss der Synthesezyklen des erfindungsgemäßen Verfahrens hergestellt werden. Die Herstellung von pharmazeutisch tolerierbaren Salzen von Peptiden ist dem Fachmann bekannt. Ein bevorzugtes pharmazeutisch tolerierbares Salz ist Acetat.

Der GLP-1-Agonist ist vorzugsweise ausgewählt aus der Gruppe bestehend aus Exendin-4, Analoga und Derivaten von Exendin-4 und pharmakologisch tolerierbaren Salzen davon.

Ein weiterer bevorzugter GLP-1-Agonist ist ein Analogon von Exendin-4 ausgewählt aus einer Gruppe bestehend aus:
H-desPro³⁶-Exendin-4-Lys₆-NH₂,
H-des(Pro^{36,37})-Exendin-4-Lys₄-NH₂,
H-des(pro^{36,37})-Exendin-4-Lys₅-NH₂ und pharmakologisch tolerierbaren Salzen davon.

Ein weiterer bevorzugter GLP-1-Agonist ist ein Analogon von Exendin-4 ausgewählt aus einer Gruppe bestehend aus:
desPro³⁶[Asp²⁸]Exendin-4 (1-39),
desPro³⁶[IsoAsp²⁸]Exendin-4 (1-39),
desPro³⁶[Met(O)¹⁴,Asp²⁸]Exendin-4 (1-39),
desPro³⁶[Met(O)¹⁴,IsoAsp²⁸]Exendin-4 (1-39),
desPro³⁶[Trp(O₂)²⁵,Asp²⁸]Exendin-2 (1-39),
desPro³⁶[Trp(O₂)²⁵,IsoAsp²⁸]Exendin-2 (1-39),
desPro³⁶[Met(O)¹⁴Trp(O₂)²⁵,Asp²⁸]Exendin-4 (1-39),
desPro³⁶[Met(O)¹⁴Trp(O₂)²⁵,IsoAsp²⁸]Exendin-4(1-39) und pharmakologisch tolerierbaren Salzen davon.

Ein weiterer bevorzugter GLP-1-Agonist ist ein Analogon von Exendin-4 ausgewählt aus einer Gruppe wie im vorigen Absatz beschrieben, bei denen an die C-Termini der Analoga von Exendin-4 das Peptid -Lys₆-NH₂ angefügt ist.

Ein weiterer bevorzugter GLP-1-Agonist ist ein Analogon von Exendin-4 ausgewählt aus einer Gruppe bestehend aus:
H-(Lys)₆-desPro³⁶[Asp²⁸]Exendin-4(1-39)-Lys₆-NH₂,
desAsp²⁸Pro³⁶,Pro³⁷,Pro³⁸Exendin-4(1-39)-NH₂,
H-(Lys)₆-desPro³⁶,Pro³⁷,Pro³⁸[Asp²⁸]Exendin-4(1-39)-NH₂,
H-Asn-(Glu)₅desPro³⁶,Pro³⁷,Pro³⁸[Asp²⁸]Exendin-4(1-39)-NH₂,
desPro³⁶,Pro³⁷,Pro³⁸[Asp²⁸]Exendin-4(1-39)-(Lys)₆-NH₂,
H-(Lys)₆-desPro³⁶,Pro³⁷,Pro³⁸[Asp²⁸]Exendin-4(1-39)-(Lys)₆-NH₂,
H-Asn-(Glu)₅-desPro³⁶,Pro³⁷,Pro³⁸[Asp²⁸]Exendin-4(1-39)-(Lys)₆-NH₂,
H-(Lys)₆-desPro³⁶[Trp(O₂)²⁵,Asp²⁸]Exendin-4(1-39)-Lys₆-NH₂,
H-desAsp²⁸Pro³⁶,Pro³⁷,Pro³⁸[Trp(O₂)²⁵]Exendin-4(1-39)-NH₂,
H-(Lys)₆-desPro³⁶,Pro³⁷,Pro³⁸[Trp(O₂)²⁵,Asp²⁸]Exendin-4(1-39)-NH₂,
H-Asn-(Glu)₅-desPro³⁶,Pro³⁷,Pro³⁸[Trp(O₂)²⁵,Asp²⁸]Exendin-4(1-39)-NH₂,
desPro³⁶,Pro³⁷,Pro³⁸[Trp(O₂)²⁵,Asp²⁸]Exendin-4(1-39)-(Lys)₆-NH₂,
H-(Lys)₆-desPro³⁶,Pro³⁷,Pro³⁸[Tr_{P}(O₂)²⁵,Asp²⁸]Exendin-4(1-39)-(Lys)₆-NH₂,
H-Asn-(Glu)₅-desPro³⁶,Pro³⁷,Pro³⁸[Trp(O₂)²⁵,Asp²⁸]Exendin-4(1-39)-(Lys)₆-NH₂,
H-(Lys)₆-desPro³⁶[Met(O)¹⁴,Asp²⁸]Exendin-4(1-39)-Lys₆-NH₂,
desMet(O)¹⁴Asp²⁸Pro³⁶,Pro³⁷,Pro³⁸Exendin-4(1-39)-NH₂,
H-(Lys)₆-desPro³⁶,Pro³⁷,Pro³⁸[Met(O)¹⁴,Asp²⁸]Exendin-4(1-39)-NH₂,
H-Asn-(Glu)₅-desPro³⁶,Pro³⁷,Pro³⁸[Met(O)¹⁴,Asp²⁸]Exendin-4(1-39)-NH₂,
desPro³⁶,Pro³⁷,Pro³⁸[Met(O)¹⁴,Asp²⁸]Exendin-4(1-39)-(Lys)₆-NH₂,
H-(Lys)₆-desPro³⁶,Pro³⁷,Pro³⁸[Met(O)¹⁴,Asp²⁸]Exendin-4(1-39)-Lys₆-NH₂,
H-Asn-(Glu)₅-desPro³⁶Pro³⁷Pro³⁸[Met(O)¹⁴Asp²⁸]Exendin-4(1-39)-(Lys)₆-NH₂,
H-(Lys)₆-desPro³⁶[Met(O)¹⁴,Trp(O₂)²⁵,Asp²⁸]Exendin-4(1-39)-Lys₆-NH₂,
desAsp²⁸Pro³⁶,Pro³⁷,Pro³⁸[Met(O)¹⁴,Trp(O₂)²⁵]Exendin-4(1-39)-NH₂,
H-(Lys)₆-desPro³⁶,Pro³⁷,Pro³⁸[Met(O)¹⁴,Trp(O₂)²⁵,Asp²⁸]Exendin-4(1-39)-NH₂,
H-Asn-(Glu)₅-desPro³⁶,Pro³⁷,Pro³⁸[Met(O)¹⁴,Asp²⁸]Exendin-4(1-39)-NH₂,
desPro³⁶,Pro³⁷,Pro³⁸[Met(O)¹⁴,Trp(O₂)²⁵,Asp²⁸]Exendin-4(1-39)-(Lys)₆-NH₂,
H-(Lys)₆-desPro^{36,Pro37},Pro³⁸[Met(O)¹⁴,Trp(O₂)²⁵,Asp²⁸]Exendin-4(1-39)-(Lys)₆-NH₂,
H-Asn-(Glu)₅-desPro³⁶,Pro³⁷,Pro³⁸[Met(O)¹⁴,Trp(O₂)²⁵,Asp²⁸]Exendin-4(1-39)-(Lys)₆-NH₂ und pharmakologisch tolerierbaren Salzen davon.
Ebenso kann der GLP-1-Agonist ausgewählt sein aus GLP-1 und Analoga und Derivaten von GLP-1. Ein weiterer bevorzugter GLP-1-Agonist ist ausgewählt aus einer Gruppe bestehend aus Arg³⁴,Lys²⁶(N^{ε}(γ-glutamyl(N^{α}-hexadecanoyl)))GLP-1(7-37) [Liraglutide] und einem pharmakologisch tolerierbaren Salz davon.
Ein weiterer bevorzugter GLP-1-Agonist ist AVE0010. AVE0010 weist die Sequenz desPro³⁶Exendin-4(1-39)-Lys₆-NH₂ auf (SEQ ID NO:1). Ebenso sind pharmakologisch tolerierbare Salze von AVE0010 bevorzugt.
Der GLP-1-Agonist, z. B. das AVE0010, wird in einer Menge von 0,01 mg/ml bis 0,5 mg/ml oder 0,05 mg/ml bis 1,5 mg/ml eingesetzt.
In der vorliegenden Anmeldung umfasst der Begriff "Insulin" nicht nur unmodifizierte Insuline, sondern auch Insulinanaloga, Insulinderivate und Insulinmetabolite. Die erfindungsgemäßen Zusammensetzungen umfassen eines oder mehrere unabhängig ausgewählt aus der Gruppe bestehend aus Insulinen (z.B. unmodifizierten Insulinen), Insulinanaloga, Insulinderivaten und Insulinmetaboliten und beliebigen Kombinationen davon.

Das wenigstens eine Insulin kann unabhängig ausgewählt sein aus der Gruppe bestehend aus Rinderinsulinen, Analoga, Derivaten und Metaboliten davon, Schweineinsulinen, Analoga, Derivaten und Metaboliten davon und Humaninsulinen, Analoga, Derivaten und Metaboliten davon. Bevorzugt ist das wenigstens eine Insulin unabhängig ausgewählt aus Humaninsulinen, Analoga, Derivaten und Metaboliten davon.
Ferner kann ein erfindungsgemäßes Insulin unabhängig aus unmodifizierten Insulinen ausgewählt sein, insbesondere aus Rinderinsulinen, Schweineinsulinen und Humaninsulinen.
Das wenigstens eine Insulin kann unabhängig ausgewählt sein aus der Gruppe bestehend aus Rinderinsulinen, Schweineinsulinen und Humaninsulinen. Stärker bevorzugt ist das wenigstens eine Insulin unabhängig ausgewählt aus Humaninsulinen. Ein erfindungsgemäßes Insulin kann aus unmodifizierten Insulinen ausgewählt sein, insbesondere aus Rinderinsulinen, Schweineinsulinen und Humaninsulinen.
Erfindungsgemäße Insulinderivate sind Derivate von einem natürlich vorkommenden Insulin oder/und einem Insulinanalogon, welche durch chemische Modifizierung erhalten werden. Die chemische Modifikation kann z.B. in der Addition einer oder mehrerer bestimmter chemischer Gruppen an eine oder mehrere Aminosäuren bestehen.
Insulinanaloga, welche in EP 0 214 826, EP 0 375 437, EP 0 678 522, EP 0 419 504, WO 92/00321, EP-A 0 368 187 und WO2009/063072 beschrieben sind, können Bestandteil der erfindungsgemäßen Zusammensetzungen sein.
Ein bevorzugtes erfindungsgemäßes Insulinanalogon kann ausgewählt sein aus der Gruppe bestehend aus Gly(A21)-Arg(B31)-Arg(B32)-Humaninsulin (Insulin Glargin), Lys(B3)-Glu(B29)-Humaninsulin; Lys^{B28} Pro^{B29} Humaninsulin (Insulin Lyspro), B28 Asp-Humaninsulin (Insulin Aspart), Humaninsulin, bei dem Prolin in der Position B28 substituiert wurde durch Asp, Lys, Leu, Val oder Ala und wo in Position B29 Lys durch Pro substituiert sein kann; AlaB26-Humaninsulin; Des(B28-B30)-Humaninsulin; Des(B27)-Humaninsulin oder B29Lys(ε-tetradecanoyl),des(B30)-Humaninsulin (Insulin Detemir), N^{εB29}-tetradecanoyl des(B30)Humaninsulin, N^{εB21}-(N^{α}-(HOOC(CH₂)₁₄CO)-γ-Glu) des B30 Humaninsulin, Lys^{B29}(N^{ε} lithocholyl-γ-Glu)-des(B30) Humaninsulin, N^{εB29}-ω-carboxypentadecanoyl-γ-L-glutaylamide desB30 Humaninsulin und N^{εB29}-ω-carboxypentadecanoyl-γ-amino-butanoyl des(B30) Humaninsulin.
Ein bevorzugtes erfindungsgemäßes Insulinderivat kann ausgewählt sein aus der Gruppe bestehend aus B29-N-myristoyl-des(B30) Humaninsulin, B29-N-palmitoyl-des(B30) Humaninsulin, B29-N-myristoyl Humaninsulin, B29-N-palmitoyl Humaninsulin, B28-N-myristoyl Lys^{B28}Pro^{B29} Humaninsulin, B28-N-palmitoyl-Lys^{B28}Pro^{B29} Humaninsulin, B30-N-myristoyl-Thr^{B29}Lys^{B30} Humaninsulin, B30-N-palmitoyl-Thr^{B29}Lys^{B30} Humaninsulin, B29-N-(N-palmitoyl-Y-glutamyl)-des(B30) Humaninsulin, B29-N-(N-lithocholyl-Y-glutamyl)-des(B30) Humaninsulin, B29-N-(ω-carboxyheptadecanoyl)-des(B30) Humaninsulin und B29-N-(ω-carboxyheptadecanoyl) Humaninsulin, N^{εB29}-tetradecanoyl des(B30)Humaninsulin, N^{εS29}-(N^{α}-(HOOC(CH₂)₁₄CO)-γ-Glu) des B30 Humaninsulin, Lys^{B29}(N^{ε} lithocholyl-γ-Glu)-des(B30) Humaninsulin, N^{εB29}-ω-carboxypentadecanoyl-γ-L-glutaylamide desB30 Humaninsulin und N^{εB29}-ω-carboxypentadecanoyl-γ-amino-butanoyl des(B30) Humaninsulin.
Ein stärker bevorzugtes erfindungsgemäßes Insulinderivat wird ausgewählt aus der Gruppe bestehend aus Gly(A21)-Arg(B31)-Arg(B32)-Humaninsulin, Lys^{B28}Pro^{B29} Humaninsulin (Insulin Lyspro), B28 Asp Humaninsulin (Insulin Aspart), B29Lys(ε-tetradecanoyl),desB30 Humaninsulin (Insulin Detemir).

Die erfindungsgemäßen Zusammensetzungen enthalten 240-3000 nmol/ml eines Insulins wie hierin definiert. Eine Konzentration von 240-3000 nmol/ml entspricht je nach verwendetem Insulin etwa einer Konzentration 1,4-35 mg/ml oder 40-500 Einheiten/ml.

Eine besonders bevorzugter erfindungsgemäßer Gegenstand ist eine Zusammensetzung wie hierin beschrieben umfassend wenigstens ein Insulin unabhängig ausgewählt aus Lys^{B28}Pro^{B29} Humaninsulin (Insulin Lyspro), B28 Asp Humaninsulin (Insulin Aspart), B29Lys(ε-tetradecanoyl), desB30 Humaninsulin (Insulin Detemir), und Insulin Glargin (Gly(A21)-Arg(B31)-Arg(B32)-Humaninsulin), und umfassend AVE0010 oder/und ein pharmakologisch tolerierbares Salz davon. Ein weiterer besonders bevorzugter Gegenstand ist eine Zusammensetzung wie hierin beschrieben umfassend Insulin Glargin (Gly(A21)-Arg(B31)-Arg(B32)-Humaninsulin) und AVE0010 (desPro³⁶Exendin-4(1-39)-Lys₆-NH₂) oder/und ein pharmakologisch tolerierbares Salz davon. Diese besonders bevorzugten Zusammensetzungen weisen vorzugsweise einen sauren pH von 1 - 6,8, stärker bevorzugt pH 3,5 - 6,8, noch stärker bevorzugt pH 3,5 - 4,5.

In einer besonderen Ausführungsform umfasst die erfindungsgemäße Formulierung die folgenden Bestandteile:
(a) desPro³⁶Exendin-4(1-39)-Lys₆-NH₂,
(b) Gly(A21)-Arg(B31)-Arg(B32)-Humaninsulin
(c) Zinkchlorid,
(d) m-Kresol,
(e) L-Methionin,
(f) Glycerin,
(g) Salzsäure, soweit zur Einstellung eines pH-Wertes von etwa 4,5 erforderlich,
(h) NaOH-Lösung, soweit zur Einstellung eines pH-Wertes von etwa 4,5 erforderlich, und
(i) Wasser.

Insbesondere besteht die erfindungsgemäße Formulierung aus den genannten Bestandteilen (a) bis (i). Gegebenenfalls kann auf m-Kresol verzichtet werden. Insoweit besteht die erfindungsgemäße Formulierung dann aus Bestandteilen (a) bis (c) und (e) bis (i).

Ein weiterer erfindungsgemäßer Gegenstand ist eine Kombination von wenigstens zwei erfindungsgemäßen Formulierungen. Hierbei werden eine erste, eine zweite Zusammensetzung und gegebenenfalls wenigstens eine weitere pharmazeutische Zusammensetzung bereitgestellt, welche jeweils das Insulin und den GLP-1-Agonisten enthalten.

Daher ist ein erfindungsgemäßer Gegenstand eine Kombination umfassend eine erste pharmazeutische Zusammensetzung und eine zweite pharmazeutische Zusammensetzung und gegebenenfalls wenigstens eine weitere pharmazeutische Zusammensetzung, welche jeweils wenigstens ein Insulin und wenigstens einen GLP-1-Agonisten umfassen und das wenigstens eine Insulin oder/und den wenigstens einen GLP-1-Agonisten in unterschiedlichen Gewichtsanteilen bezogen auf das Gesamtgewicht der Zusammensetzung enthalten.

In der vorliegenden Anmeldung bedeutet "gegebenenfalls wenigstens eine weitere pharmazeutische Zusammensetzung", dass die erfindungsgemäße Kombination neben der ersten und der zweiten pharmazeutischen Zusammensetzung wenigstens eine weitere pharmazeutische Zusammensetzung umfassen kann. Die erfindungsgemäße Kombination kann also z. B. 3, 4, 5, 6, 7, 8, 9, 10 oder mehr erfindungsgemäße pharmazeutische Zusammensetzungen umfassen.

Bevorzugt sind Kombinationen, welche eine erste und eine zweite erfindungsgemäße pharmazeutische Zusammensetzung enthalten.

Ebenso sind Kombinationen bevorzugt, welche eine erste, eine zweite und eine dritte erfindungsgemäße pharmazeutische Zusammensetzung enthalten.

Ebenso sind Kombinationen bevorzugt, welche eine erste, eine zweite, eine dritte und eine vierte erfindungsgemäße pharmazeutische Zusammensetzung enthalten.

Ebenso sind Kombinationen bevorzugt, welche eine erste, eine zweite, eine dritte, eine vierte und eine fünfte pharmazeutische Zusammensetzung enthalten.

Die Gewichtsanteile des wenigstens einen Insulins und des wenigstens einen GLP-1-Agonisten können in der ersten pharmazeutischen Zusammensetzung, der zweiten pharmazeutischen Zusammensetzung und der gegebenenfalls wenigstens einen weiteren pharmazeutischen Zusammensetzung so ausgewählt werden, dass die pharmazeutischen Zusammensetzungen unterschiedliche Verhältnisse von Insulin zum GLP-1-Agonisten bezogen auf den Gewichtsanteil enthalten.

Hierbei kann die erste Zusammensetzung das kleinste Verhältnis und die zweite Zusammensetzung das nächst größere Verhältnis enthalten. Sofern wenigstens eine weitere Zusammensetzung vorhanden ist, kann diese das nächst größere Verhältnis enthalten. Sofern noch eine weitere Zusammensetzung vorhanden ist, kann diese das wiederum nächst größere Verhältnis enthalten. Die Zusammensetzungen können also von der ersten zur zweiten und gegebenenfalls weiteren Zusammensetzungen ansteigende Verhältnisse von Insulin zum GLP-1-Agonisten bezogen auf den Gewichtsanteil enthalten.

Der Gewichtsanteil von einem der beiden Wirkstoffe, also des wenigstens einen Insulins oder des wenigstens einen GLP-Agonisten, in der ersten pharmazeutischen Zusammensetzung, der zweiten pharmazeutischen Zusammensetzung und der gegebenenfalls wenigstens einen weiteren pharmazeutischen Zusammensetzung wird bevorzugt jeweils so ausgewählt, dass durch Verabreichung eines bestimmten Volumens der ersten, der zweiten oder/und der wenigstens einen weiteren Zusammensetzung die vorbestimmte Dosis dieses Wirkstoffs verabreicht werden kann. Besonders bevorzugt ist dieser Wirkstoff das wenigstens eine Insulin.

Der Gewichtsanteil des anderen der beiden Wirkstoffe, also des wenigstens einen Insulins oder des wenigstens einen GLP-1-Agonisten, in der ersten pharmazeutischen Zusammensetzung, der zweiten pharmazeutischen Zusammensetzung und der gegebenenfalls wenigstens einen weiteren pharmazeutischen Zusammensetzung wird bevorzugt so ausgewählt, dass die Verhältnisse von Insulin zum GLP-1-Agonisten bezogen auf den Gewichtsanteil von der ersten zur zweiten und gegebenenfalls weiteren Zusammensetzungen ansteigen. Besonders bevorzugt ist dieser Wirkstoff der wenigstens eine GLP-1-Agonist.

Darüber hinaus wird der Gewichtsanteil des anderen der beiden Wirkstoffe in den pharmazeutischen Zusammensetzungen so bestimmt, dass eine der pharmazeutischen Zusammensetzungen so ausgewählt werden kann, dass die zu verabreichende Dosis des ersten der beiden Wirkstoffe und die zu verabreichende Dosis des zweiten Wirkstoffs in einem bestimmten Volumen gegeben sind. Damit wird eine pharmazeutische Zusammensetzung ausgewählt, welche das gewünschte Verhältnis enthält.

Theoretisch könnte für jedes einzelne therapeutisch gewünschte Verhältnis der Gewichtsanteile vom wenigstens einen Insulin zum wenigstens einen GLP-1-Agonisten eine pharmazeutische Zusammensetzung bereitgestellt werden, um eine optimale bedarfsgerechte Dosierung für beide Wirkstoffe für jeden Patienten zu erzielen.

In der vorliegenden Erfindung ist eine bestimmte Anzahl von pharmazeutischen Zusammensetzungen ausreichend, um die in der Praxis notwendigen Dosierungen für beide Wirkstoffe zu erfassen. Es wird für jeden Patienten ein bestimmter Dosierungsbereich innerhalb eines therapeutisch sinnvollen Intervalls für jeden der beiden Wirkstoffe bestimmt. Die zu verabreichende Dosis soll hierbei für einen bestimmten Patienten im Wesentlichen innerhalb dieses Dosierungsbereiches schwanken, ohne dass eine Über- oder eine Unterdosierung vorliegt.

Da primär die Insulinmenge an den einzelnen Patienten angepasst und präzise dosiert werden muss, ermöglicht es der Konzentrationsbereich vom GLP-1-Agonisten, dass eine erfindungsgemäße pharmazeutische Zusammensetzung, welche ein bestimmtes Verhältnis von wenigstens einem Insulin zu dem wenigstens einem GLP-1-Agonisten enthält, einen therapeutischen Bereich von Insulindosierungen gleichzeitig mit der zugehörigen synergistischen GLP-1-Agonistenmenge abdeckt. Das Verhältnis kann so ausgewählt werden, dass zu jeder gewünschten Insulindosierung eine Dosierung des wenigstens einen GLP-1-Agonisten korrespondiert, welche innerhalb des gewünschten Bereichs, z.B. des synergistischen Bereichs, liegt. Wie weiter oben ausgeführt, können die Verhältnisse der ersten, zweiten und gegebenenfalls wenigstens einen weiteren Zusammensetzung des Arzneimittels ferner so gewählt werden, dass die Verhältnisse von der ersten zur zweiten und der gegebenenfalls wenigstens einen weiteren Zusammensetzung ansteigen. Ist die Dosierung des GLP-1-Agonisten bei der gewünschten Insulindosierung einer Zusammensetzung (z. B. der ersten Zusammensetzung) außerhalb (in der Regel oberhalb) des gewünschten Dosierungsbereiches des GLP-1-Agonisten, so wird die nächste Zusammensetzung (z.B. die zweite Zusammensetzung) oder eine weitere Zusammensetzung mit einem größeren Verhältnis des wenigstens einen Insulins zu dem wenigstens einen GLP-1-Agonisten zur Anwendung ausgewählt, in welcher die Menge des GLP-1-Agonisten bei der gewünschten Insulindosis im gewünschten Bereich liegt. Die Verhältnisse der ersten, zweiten und gegebenenfalls wenigstens einen weiteren Zusammensetzung der Kombination können ferner so gewählt werden, dass die Bereiche der Insulindosierungen, welche zu den gewünschten Dosierungen des wenigstens einen GLP-1-Agonisten korrespondieren, aneinander anschließen oder/und einander überlappen. Bevorzugt überlappen die Bereiche. Überlappung bedeutet insbesondere, dass wenigstens zwei Zusammensetzungen ausgewählt werden können, welche bei der gewünschten Dosierung des wenigstens einen Insulins je eine Menge des wenigstens einen GLP-1-Agonisten enthalten, welche innerhalb des gewünschten Dosierungsbereichs liegt.

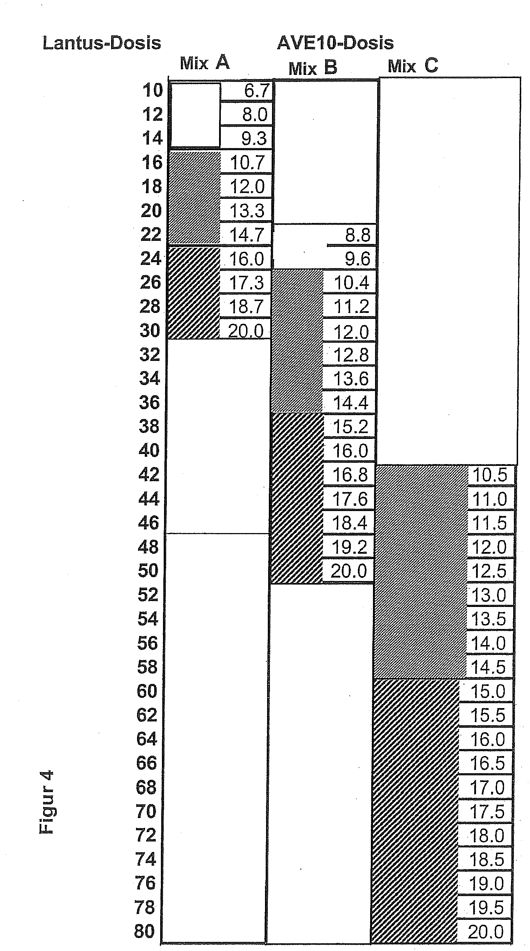

Beispielsweise genügen 3 Zusammensetzungen, um die Dosis des wenigstens einen Insulins für einen individuellen Patienten auf einen Wert ausgewählt aus dem Bereich von 15 bis 80 Einheiten Insulin einzustellen und gleichzeitig den GLP-1-Agonisten mit einer Menge innerhalb des Bereichs von 10 bis 20 µg zu dosieren (siehe Figur 4).

Ebenso kann eine erfindungsgemäße Kombination bereitgestellt werden, in welchem das Verhältnis so ausgewählt wird, dass zu jeder gewünschten Dosierung des GLP-1-Agonisten eine Dosierung des wenigstens einen Insulins korrespondiert, welche innerhalb des gewünschten Bereichs liegt. Die Verhältnisse der ersten, zweiten und gegebenenfalls wenigstens einen weiteren Zusammensetzung des Arzneimittels können ferner so gewählt werden, dass die Bereiche der Dosierungen des GLP-1-Agonisten, welche zu den gewünschten Dosierungen des wenigstens einen Insulins korrespondieren, aneinander anschließen oder/und einander überlappen. Bevorzugt überlappen die Bereiche. Überlappung bedeutet in diesem Zusammenhang insbesondere, dass wenigstens zwei Zusammensetzungen ausgewählt werden können, welche bei der gewünschten Dosierung des wenigstens einen GLP-1-Agonisten je eine Menge des wenigstens einen Insulins enthalten, welche innerhalb des gewünschten ' Dosierungsbereichs liegt.

Vorzugsweise enthält die erfindungsgemäße Kombination maximal 10 pharmazeutische Zusammensetzungen wie oben definiert, stärker bevorzugt maximal 5, maximal 4, maximal 3 oder 2 pharmazeutische Zusammensetzungen.

Die erfindungsgemäßen Zusammensetzungen können den wenigstens einen GLP-1-Agonisten in jeweils identischen oder verschiedenen Gewichtsanteilen enthalten. Beispielsweise können mindestens zwei der erfindungsgemäßen Zusammensetzungen den wenigstens einen GLP-1-Agonisten in einem im wesentlichen identischen Gewichtsanteil enthalten.

Es ist bevorzugt, dass die erste, zweite und die gegebenenfalls weitere(n) Zusammensetzung(en) den wenigstens einen GLP-1-Agonisten in einem im wesentlichen identischen Gewichtsanteil enthalten und das wenigstens eine Insulin in unterschiedlichen Gewichtsanteilen enthalten.

Die erfindungsgemäßen Zusammensetzungen können aber auch das wenigstens eine Insulin in jeweils identischen oder verschiedenen Gewichtsanteilen enthalten. Beispielsweise können mindestens zwei der erfindungsgemäßen Zusammensetzungen das wenigstens eine Insulin in einem im wesentlichen identischen Gewichtsanteil enthalten.

Es ist besonders bevorzugt, dass die erste, zweite und die gegebenenfalls weitere(n) Zusammensetzung(en) das wenigstens eine Insulin in einem im wesentlichen identischen Gewichtsanteil enthalten und den wenigstens einen GLP-1-Agonisten in unterschiedlichen Gewichtsanteilen enthalten.

Eine erste bevorzugte erfindungsgemäße Zusammensetzung umfasst:
(a) AVE0010 etwa 0,025 mg
(b) Insulin Glargin etwa 3,64 mg
(c) Zinkchlorid etwa 0,06 mg
(d) 85%iges Glycerin etwa 20,0 mg
(e) m-Kresol etwa 2,7 mg
(f) L-Methionin etwa 3,0 mg
(g) NaOH q.s.pH 4,5
(h) HCl 36% q.s.pH 4,5
(i) Wasser ad 1 mL

Eine zweite bevorzugte erfindungsgemäße Zusammensetzung umfasst:
(a) AVE0010 etwa 0,04 mg
(b) Insulin Glargin etwa 3,64 mg
(c) Zinkchlorid etwa 0,06 mg
(d) 85%iges Glycerin etwa 20,0 mg
(e) m-Kresol etwa 2,7 mg
(f) L-Methionin etwa 3,0 mg
(g)NaOH q.s.pH 4,5
(h)HCl 36% q.s.pH 4,5
(i) Wasser ad 1 mL

Eine dritte bevorzugte erfindungsgemäße Zusammensetzung umfasst:
(a) AVE0010 etwa 0,066 mg
(b) Insulin Glargin etwa 3,64 mg
(c) Zinkchlorid etwa 0,06 mg
(d) 85%iges Glycerin etwa 20,0 mg
(e) m-Kresol etwa 2,7 mg
(f) L-Methionin etwa 3,0 mg
(g) NaOH q.s.pH 4,5
(h) HCl 36% q.s.pH 4,5
(i) Wasser ad 1 mL

Eine vierte bevorzugte erfindungsgemäße Zusammensetzung umfasst:
(a) AVE0010 etwa 0,1 mg
(b) Insulin Glargin etwa 3,64 mg
(c) Zinkchlorid etwa 0,06 mg
(d) 85%iges Glycerin etwa 20,0 mg
(e) m-Kresol etwa 2,7 mg
(f) L-Methionin etwa 3,0 mg
(g) NaOH q.s.pH 4,5
(h) HCl 36% q.s.pH 4,5
(i) Wasser ad 1 mL

Besonders bevorzugt ist eine Kombination umfassend mindestens 2, 3 oder 4 der genannten ersten, zweiten, dritten und vierten bevorzugten Zusammensetzung.

"Etwa" bedeutet in der vorliegenden Anmeldung, dass die Bestandteile z. B. innerhalb der Bereiche von ±10, ±20, oder ±30 um die angegebenen Zahlenwerte in den erfindungsgemäßen Zusammensetzungen oder/und Kombinationen vorhanden sein können; bevorzugt ist ±10.

Sofern die erfindungsgemäße Zusammensetzung oder Kombination mehr als ein Insulin umfasst, so werden diese Insuline unabhängig voneinander ausgewählt.

Sofern die erfindungsgemäße Zusammensetzung oder Kombination mehr als einen GLP-1-Agonisten umfasst, so werden diese GLP-1-Agonisten unabhängig voneinander ausgewählt.

Die erfindungsgemäße Kombination wird insbesondere als Arzneimittel bereitgestellt.

Ein weiterer erfindungsgemäßer Gegenstand ist ein Kit umfassend eine erfindungsgemäße Kombination umfassend mindestens eine, maximal vier, erfindungsgemäße Zusammensetzung(en) sowie ggf. Lantus®. Der erfindungsgemäße Kit kann für die Anwendung durch medizinisches Personal oder durch medizinische Laien, insbesondere den Patienten selbst oder Hilfspersonen wie Angehörige bestimmt sein. Im erfindungsgemäßen Kit sind die einzelnen pharmazeutischen Zusammensetzungen, welche die erfindungsgemäße Kombination umfasst, in getrennten Verpackungen zusammengefasst, so dass der Patient die jeweils an den aktuellen Bedarf angepasste Zusammensetzung auswählen und eine bedarfsgerechte Menge verabreichen kann. Der erfindungsgemäße Kit umfasst zum Beispiel die erfindungsgemäße Kombination in Form eines Satzes von Spritzen, Glasampullen oder/und Stiften, welche mindestens eine der erfindungsgemäßen Zusammensetzungen, ggf. in Kombination mit der Zusammensetzung von Lantus® enthalten.

Eine geeignete Verpackung ist eine Spritze oder ein Glasgefäß mit einem geeigneten Verschluß, aus denen bei Bedarf einzelne therapeutisch wirksame Dosen entnommen werden können. Ebenso geeignet sind Injektionsstifte ("Stifte", "Pens") zur Verabreichung von Insulin, welche einen Behälter (z. B. eine Patrone) umfassen, welcher eine erfindungsgemäße pharmazeutische Zusammensetzung enthält.

Insbesondere ist der erfindungsgemäße Kit ein Injektionsstift bestehend aus zwei getrennten Behältern, aus denen bei Bedarf jeweils einzelne therapeutische Dosen entnommen werden können. Ebenso ist der Kit eine Spritze, in der bestehend aus
zwei Behältern, in der der zweite Behälter als Reservoir needle (Reservoir Nadel) ausgestattet ist.

In der vorliegenden Erfindung besteht der Kit vorzugsweise aus einer Kombination aus einer ersten Formulierung, die den GLP-1 Agonisten, ein Insulin, Glycerol, Zinkchlorid, ggf. m-Kresol, L-Methionin bei einem pH-Wert von 4,5 in Wasser enthält und einer zweiten Formulierung, die bevorzugt ein Insulin, Glycerol, Zinkchlorid und m-Kresol bei einem pH Wert von 4,5 in Wasser enthält.

Die erste Formulierung kann bevorzugt folgende Zusammensetzung haben:

| | | |
|---|---|---|
| (a) | AVE0010 | etwa 0,4 mg oder etwa 0,8 mg |
| (b) | Insulin Glargin | etwa 3,64 mg |
| (c) | Zinkchlorid | etwa 0,06 mg |
| (d) | 85 %iges Glycerol | etwa 20,0 mg |
| (e) | m-Kresol | 0,0 mg oder etwa 2,7 mg |
| (f) | L-Methionin | etwa 3,0 mg |
| (g) | NaOH | q.s.pH 4,5 |
| (h) | HCl 36% | q.s.pH 4,5 |
| (i) | Wasser | ad 1 ml. |

Die zweite Formulierung kann bevorzugt folgende Zusammensetzung haben:

| | | |
|---|---|---|
| (a) | Insulin Glargin | etwa 3,64 mg |
| (b) | Zinklchlorid | etwa 0,06 mg |
| (c) | 85 %iges Glycerol | etwa 20,0 mg |
| (d) | m-Kresol | etwa 2,7 mg |
| (e) | NaOH | q.s.pH 4,5 |
| (f) | HCl 36 % | q.s.pH 4,5 |
| (g) | Wasser | ad 1 ml. |

Weiterhin beschrieben wird ein Verfahren zur Behandlung eines Patienten mit einer erfindungsgemäßen Zusammensetzung, umfassend Verabreichen der Zusammensetzung an den Patienten. Ebenfalls beschrieben wird ein Verfahren zur Behandlung eines Patienten mit einer erfindungsgemäßen Kombination oder mit einem Kit wie hierin beschrieben. Insbesondere umfasst dieses Verfahren die Verabreichung einer erfindungsgemäßen Kombination umfassend eine erste pharmazeutische Zusammensetzung und eine zweite pharmazeutische Zusammensetzung und gegebenenfalls wenigstens eine weitere pharmazeutische Zusammensetzung, welche jeweils wenigstens ein Insulin und wenigstens einen GLP-1-Agonisten umfassen und das wenigstens eine Insulin oder/und den wenigstens einen GLP-1-Agonisten in unterschiedlichen Gewichtsanteilen bezogen auf das Gesamtgewicht der Zusammensetzung enthalten, wobei das Verfahren umfasst:
(a) Auswählen einer Dosis des wenigstens einen Insulins, welche verabreicht werden soll,
(b) Auswählen einer Dosis des wenigstens einen GLP-1-Agonisten, welche verabreicht werden soll,
(c) Auswählen einer Zusammensetzung aus der ersten, zweiten und gegebenenfalls wenigstens einen weiteren Zusammensetzung des Arzneimittels, welche die Dosen aus (a) und (b) in einer Konzentration enthält, so dass die Dosen aus (a) und (b) in gleichem Volumen vorliegen, und
(d) Bestimmen und Verabreichen einer Menge, welche den Dosen aus (a) und (b) entspricht.
Die Bestimmung der Dosis nach Schritt (a) oder/und Schritt (b) erfolgt nach dem individuellen Bedarf der Patienten.

Schritt (c) des Behandlungsverfahrens kann anhand einer Tabelle durchgeführt wird. Diese Tabelle kann Bestandteil der erfindungsgemäßen Kombination, des erfindungsgemäßen Arzneimittels oder des erfindungsgemäßen Kits sein. Beispiel 2 enthält ein Beispiel für eine erfindungsgemäße Tabelle.

Die erfindungsgemäße Zusammensetzung, die erfindungsgemäße Kombination, das erfindungsgemäßen Arzneimittel oder/und das erfindungsgemäße Kit ist insbesondere zur Behandlung von Diabetes mellitus vorgesehen, insbesondere zur Behandlung von Diabetes mellitus Typ I oder Typ II. Weitere mögliche Indikationen sind Symptome, welche mit Diabetes mellitus assoziiert sind. Bevorzugt wird die erfindungsgemäße Zusammensetzung zur Einstellung der nüchternen, der postprandialen oder/und der postabsorptiven Plasmaglucosekonzentration, zur Verbesserung der Glucosetoleranz, zur Prävention einer Hypoglykämie, zur Prävention eines Funktionsverlustes der β-Zellen des Pankreas, zur Gewichtsabnahme oder/und zur Prävention einer Gewichtszunahme eingesetzt. Weiterhin beschrieben wird ein Verfahren zur Herstellung einer erfindungsgemäßen Zusammensetzung, einer erfindungsgemäßen Kombination, oder/und eines erfindungsgemäßen Kits umfassend Formulieren eines GLP-1-Agonisten oder/und eines pharmakologisch tolerierbares Salzes davon mit einem Insulin oder/und einem pharmazeutisch akzeptablen Salz davon, Methionin und gegebenenfalls mindestens einem pharmazeutisch akzeptablen Hilfsstoff. Ebenfalls beschrieben wird ein Verfahren zur Herstellung einer erfindungsgemäßen Zusammensetzung umfassend Formulieren eines GLP-1-Agonisten oder/und eines pharmakologisch tolerierbares Salzes davon mit Methionin und gegebenenfalls mindestens einem pharmazeutisch akzeptablen Hilfsstoff. Weiterhin beschrieben wird die Verwendung der erfindungsgemäßen Zusammensetzungen zusammen mit der Verabreichung von Metformin, Insulin Glargin oder AVE0010, insbesondere in einer add-on Therapie zur Verabreichung von Metformin, Insulin Glargin oder AVE0010.
Insbesondere enthält die Zusammensetzung des Pro³⁶ Exendin-4(1-39)-Lys₆-NH₂ (AVE0010) und/oder ein pharmakologisch tolerierbares Salz davon, Insulin Glargin und/oder ein pharmakologisch tolerierbares Salz davon.
Besonders bevorzugt ist die add-on Therapie der bevorzugten Zusammensetzung in Diabetes Typ II Patienten, die nicht hinreichend mit Insulin Glargin und/oder AVE0010 kontrolliert werden können. Es sind auch Patienten betroffen, die jünger sind als 50 Jahre und/oder einen Bodymaß Index von mindestens 30 haben. Die add-on Therapie beinhaltet insbesondere die Behandlung von Diabetes Typ II mit der erfindungsgemäßen Zusammensetzung als Zusatz zu Metformin, AVE0010 und/oder Insulin Glargin. Die erfindungsgemäße Zusammensetzung kann in einem Zeitintervall von 24 h zugesetzt werden (once-a-day Dosierung). Metformin, Insulin Glargin und AVE0010 können verabreicht werden mittels verschiedener Verabreichungswege. Metformin kann oral verabreicht werden, AVE0010 und Insulin Glargin jeweils subkutan.

Die Patienten, die mit der beschriebenen add-on Therapie behandelt wurden können einen HbA1c Wert von 7 % bis 10 % haben. Sie haben bevorzugt ein Alter von 18 bis 50 Jahren.

Die Verwendung in der add-on Therapie ist insbesondere anwendbar an Patienten, bei denen Diabetes Typ II nicht hinreichend mit Metformin, AVE0010 oder Insulin Glargin alleine kontrolliert werden kann. Bevorzugt ist die Therapie bei nicht hinreichender Kontrolle durch Insulin Glargin oder AVE0010. Weiterhin beschrieben wird die Verwendung der erfindungsgemäßen Zusammensetzung, als Zusatz zu einer Diät, um den Blutzuckerspiegel in Typ II Diabetes Patienten zu kontrollieren, wenn die Anwendung von Insulin Glargin und AVE0010 angezeigt ist.

Insbesondere wird Metformin mindestens 1,0 g/Tag, bevorzugt mindestens 1,5 g/Tag für 3 Monate verabreicht.

Die Erfindung wird weiterhin durch die folgenden Abbildungen und Beispiele erläutert.

Figur 1 zeigt den Gehalt an oxidiertem Methionin Met(ox) in AVE0010 nach 1 Monat Lagerung bei unterschiedlichen Temperaturen relativ zum Ausgangszeitpunkt. Der Rahmen zeigt die Werte für die AVE0010-Referenzformulierung Nr. 1 und 2.

Figur 2 zeigt den Gehalt an Verunreinigungen von AVE0010 ohne Met(ox) nach 1 Monat Lagerung bei unterschiedlichen Temperaturen relativ zum Ausgangszeitpunkt.

Die Rahmen zeigen die Werte der AVE0010-Referenzformuiierungen bei 25° C bzw. bei 40°C.

Figur 3 zeigt den Gehalt an Verunreinigungen von Insulin Glargin nach 1 Monat Lagerung bei unterschiedlichen Temperaturen relativ zum Ausgangszeitpunkt. Die schmalen Rahmen zeigen die Werte der Insulin-Glargin-Referenzformulierungen bei 25° C bzw. bei 40°C. Die breiten Rahmen kennzeichnen die Formulierungen mit den geringsten Anteilen an AVE0010-Verunreinigungen.

Figur 4: Das "3 pens cover all"-Konzept.

### Beispiel 1

### 1. Gegenstand der Untersuchung

Es wurde die physikalische und chemische Stabilität von Zusammensetzungen umfassend einen GLP-1-Agonisten (AVE0010) und eines Insulins (Insulin Glargin, Lantus) getestet.

### 2. Eingesetzte Formulierungen

Für die getesteten Formulierungen wurden die Substanzen in folgenden Konzentrationen/Mengen eingesetzt.

| Substanz | Arzneibuch | Hersteller | Bezeichnung | eingesetzte Menge [mg/mL] |
|---|---|---|---|---|
| Insulin glargine | | Sanofi-Aventis | | 3,63 |
| | | | | 7,27 |
| | | | | 10,67 |
| AVE0010 | | Poly Peptide LabTorrance CA, USA | | 0,1 |
| | | | | 0,025 |
| Methionin | USP | MP Biomedicals | | 3 |
| Zinkchlorid | Ph. Eur., USP, BP | Merck | | 0,03 |
| | | | | 0,06 |
| | | | | 0,09 |
| Glycerin 85 % | Ph. Eur., JP | Hedinger, Stuttgart | | 20 |
| | | | | 18 |
| m-Kresol | Ph. Eur., USP | Hedinger, Stuttgart | | 2,7 |
| Polysorbate 20 | Ph.Eur.,JP | Kolb | Tween 20 | 0,02 |
| Polysorbate 80 | Ph. Eur. | SEPPIC | Tween 80 | 0,02 |
| Poloxamer 188 | | BASF, Ludwigshafen | Lutrol F68 | 0,02 |
| Benzalkoniumchlorid | Ph. Eur., JP | Sigma-Aldrich | | 0,02 |
| L-Lysine | | Resum, F-Ham, Degussa | | 1,0 |
| | | | | 5,0 |
| Acetat | | | | 1,75 |
| | | | | 3,5 |
| NaOH | Ph. Eur,, JP | Merck | | 0,1 N, zur Einstellung des pH 4,0 oder 4,5 |
| HCl | Ph. Eur., JP | Merck | | 0,1 N, zur Einstellung des pH 4,0 oder 4,5 |
| Wfl | | | | Ad 1 mL |

Sofern in Zusammenhang mit einem Bestandteil einer Formulierung ein Faktor vermerkt ist (z.B. 1/2, 1/4, 2x, 3x, 5x, wie in 1/2 Acetat, 5x Lysin, 2x Lantus und 3x Lantus), wurden die Konzentrationen der betreffenden Substanz in einer um den Faktor verringerten oder vermehrten Konzentration eingesetzt.

### 3. Testverfahren

### 3.1 Physikalische Stabilität

### 3.1.1 THT-Test

Thioflavin T (THT) bindet spezifisch an Proteinfibrillen, was zu einer Veränderung der THT-Fluoreszenz führt. THT bindet nicht an AVE0010 oder Insulin. Die Kinetik der Fibrillenbildung kann in Anwesenheit von THT als Veränderung der Fluoreszenz gemessen werden. Ein Anstieg der Fluoreszenz entspricht einer Fibrillenbildung. Die Form der Kurven erlaubt Schlüsse über die Tendenz einer Formulierung, Fibrillen zu bilden.

Fluoreszenzmessungen wurden auf einem Tecan Infinite 200 Fluoreszenzmessgerät durchgeführt. Zur Analyse der Fibrillationskinetik wurde ein Photomed FluoDia 770 Hochtemperatur-Fluoreszenz-Mikroplattenleser verwendet. Die Thioflavin-T-Fluoreszenzspektren wurde mit einem Tecan Infinite 200 Fluoreszenzmessgerät bei 23 °C durchgeführt. 900 µl Insulin wurden mit 10 µl Thioflavin T (1 mM in H₂O) gemischt. Die Mischung wurde dann in eine schwarze V-förmige 96-Lochplatte von Biozym (100 µl pro Loch) verteilt. Die Fluoreszenzemission wurde zwischen 470 und 600 nm (Schrittweite 1 nm) nach Anregung bei 450 nm mit einer Verstärkung von 100, einer Integrationszeit von 200 µs und 25 Ablesungen bei Raumtemperatur gemessen.

Die Bindekinetik von Thioflavin T wurde auf einem Photomed FluoDia 770 Hochtemperatur-Fluoreszenz-Mikroplattenleser gemessen. Das Instrument besteht im Wesentlichen aus einer 50W-Quarzhalogenlampe zur Anregung, Filterrädern für die Anregung und Emission, welche jeweils bis zur 4 Filtersets enthalten können, und einen PMT-Detektor. Die Heizplatte für 96-Lochplatten erlaubt eine sehr hohe Temperaturgenauigkeit (besser als ±0,3 °C).

10 µl einer Lösung von Thioflavin T (10,1 mM in ultrareinem Wasser) wurden zu 1 ml der Formulierungen gegeben und leicht gemischt, indem die Röhrchen mehrere Male umgedreht wurden. Die Mischung wurde dann in eine schwarze V-förmige 96-Lochplatte von Biozym (100 µl pro Loch, 8 Löcher pro Probe) verteilt. Alle Messungen wurden mit den folgenden Parametern durchgeführt:

| | |
|---|---|
| Anzahl der Zyklen: | 181 |
| Anregungsfilter: | 450 nm |
| Intervall: | 1 min |
| Emissionsfilter: | 486 nm |
| Integrationszeit: | 20 ms |
| Temperaturkontrolle: | Standard- Temperaturkontrollmodus |
| Anzahl der Mittlungen: | 4 |
| Zieltemperatur: | 70°C |
| Abschwächung: | 4 |

Fluoreszenzmittelwerte wurden aus 8 parallelen Messungen bestimmt.

### 3.2 Chemische Stabilität

Die Formulierungen wurden auf chemische Stabilität nach der Herstellung (t0) oder nach Lagerung für 1 Monat bei 4°C, 25°C (60% relative Luftfeuchte) und 40°C (75% relative Luftfeuchte) getestet. Die Messungen wurden auf einem HPLC-Gerät (Typ alliance) von Water Systems durchgeführt, wobei die 100%-Peakflächenmethode verwendet wurde. Zur Trennung wurde ein Gradient aus 0,1 % TFA und Acetonitril als mobile Phase und eine C18-Umkehrphasensäure (Jupiter) als feste Phase verwendet. Zur Analyse wurde die Formulierung mit einer Zinkacetatlösung behandelt wurde, was zur Präzipitation von Insulin Glargin führte. Die Präzipitate wurden abzentrifugiert, und es wurde nur der Überstand analysiert.

Verunreinigungen von Insulin Glargin: Die Menge der Verunreinigungen wurde mit einem HPLC (Water Systems) bestimmt, wobei die 100%-Peakflächenmethode eingesetzt wurde. Zur Trennung wurde als mobile Phase eine Natriumphosphatgepufferte Lösung (pH 2,5) mit einem NaCl- und Acetonitrilgradienten verwendet. Als stationäre Phase wurde eine C18-Umkehrphasensäure (Supersher) verwendet.

### 4. Zusammenfassung der Versuchsdaten zur physikalischen Stabilität

| Formulierung | | Zusammensetzung | pH | THT 3h, 70°C relative Fluoreszenzintensität bei 486 nm |
|---|---|---|---|---|
| Nr. | Charge | | | |
| 1 | 630 | AVE0010 Standard industrial scale | 4,5 | 536 |
| 2 | 567 | AVE0010 Standard frisch | 4 | 518 |
| 3 | 631 | Lantus Standard industrial scale | 4,0 | 2952 |
| 4 | 560 | Lantus Standard frisch | 4 | 1566 |
| 5 | 568 | Lantus form., AVE0010 | 4 | 2037 |
| 6 | 569 | Lantus form., AVE0010 1/2 Acetatpuffer | 4 | 11763 |
| 7 | 570 | Lantus form., AVE0010 Acetatpuffer | 4 | 69184 |
| 8 | 582 | Lantus form., AVE0010 Methionin | 4 | 2053 |
| 9 | 583 | Lantus form., AVE0010 1/2 Acetatpuffer Methionin | 4 | 18814 |
| 10 | 584 | Lantus form., AVE0010 Polysorbat 20 | 4 | 8183 |
| 11 | 585 | Lantus form., AVE0010 Polysorbat 20 Methionin | 4 | 6731 |
| 12 | 586 | Lantus form., AVE0010 Polysorbat 20 1/2 Acetatpuffer | 4 | 13897 |
| 13 | 587 | Lantus form., AVE0010 Polysorbat 20 1/2 Acetatpuffer Methionin | 4 | 22200 |
| 14 | 588 | Lantus form., AVE0010 Polysorbat 20 Acetatpuffer Methionin | 4 | 134093 |
| 15 | 590 | Lantus form., AVE0010 Lysin | 4 | 3362 |
| 16 | 591 | Lantus form., AVE0010 Lysin 1/2 Acetatpuffer | 4 | 19677 |
| 17 | 592 | Lantus form., AVE0010 Lysin 1/2 Acetatpuffer Polysorbat 20 | 4 | 30176 |
| 18 | 593 | Lantus form. 1/4 AVE0010 | 4 | 3107 |
| 19 | 594 | Lantus form. 1/4 AVE0010 5x Lysin | 4 | 74662 |
| 20 | 595 | 2x Lantus AVE0010 | 4 | 4504 |
| 21 | 596 | 3x Lantus AVE0010 | 4 | 30251 |
| 22 | 604 | Lantus form., AVE0010 | 4,5 | 4357 |
| 23 | 605 | Lantus form., AVE0010 1/2 Acetatpuffer | 4,5 | 36338 |
| 24 | 606 | Lantus form., AVE0010 Acetatpuffer | 4,5 | 72370 |
| 25 | 607 | Lantus form., AVE0010 Methionin | 4,5 | 5429 |
| 26 | 608 | Lantus form., AVE0010 1/2 Acetatpuffer Methionin | 4,5 | 34714 |
| 27 | 609 | Lantus form., AVE0010 Polysorbat 20 | 4,5 | 1166 |
| 28 | 610 | Lantus form., AVE0010 Polysorbat 20 Methionin | 4,5 | 5564 |
| 29 | 611 | Lantusform., AVE0010 Polysorbat 20 1/2 Acetatpuffer | 4,5 | 12115 |
| 30 | 612 | Lantus form., AVE0010 Polysorbat 20 1/2 Acetatpuffer Methionin | 4,5 | 16397 |
| 31 | 613 | Lantus form., AVE0010 Polysorbat 20 Acetatpuffer Methionin | 4,5 | 779 |
| 32 | 614 | Lantus form., AVE0010 Lysin | 4,5 | 9726 |
| 33 | 615 | Lantus form., AVE0010 Lysin 1/2 Acetatpuffer | 4,5 | 74027 |
| 34 | 616 | Lantus form., AVE0010 Lysin 1/2 Acetatpuffer Polysorbat 20 | 4,5 | 9520 |
| 35 | 617 | Lantus form. 1/4 x AVE0010 | 4,5 | 3713 |
| 36 | 618 | Lantus form. 1/4 x AVE0010 5x Lysin | 4,5 | 83384 |
| 37 | 619 | 2x Lantus AVE0010 | 4,5 | 13120 |
| 38 | 620 | 3x Lantus AVE0010 | 4,5 | 41684 |
| 39 | 657 | Lantus form. AVE0010 Polysorbat 80 Methionin | 4 | 9309 |
| 40 | 658 | Lantus form., AVE0010 Poloxamer 188 Methionin | 4 | 767 |
| 41 | 659 | Lantus form., AVE0010 Benzalkoniumchlorid Methionin | 4 | 1040 |
| 42 | 660 | Lantus form., AVE0010 Polysorbat 80 Methionin | 4,5 | 16803 |
| 43 | 661 | Lantus form., AVE0010 Poloxamer 188 Methionin | 4,5 | 689 |
| 44 | 662 | Lantus form., AVE0010 Benzalkoniumchlorid Methionin | 4,5 | 942 |

### 5. THT-Test

Methionin hat keinen Einfluß auf die Tendenz zur Fibrillenbildung. Die Formulierungen

| Nr. | Zusammensetzung | Fluoreszenzintensität bei 486 nm |
|---|---|---|
| | | |
| 2 | AVE0010 Standard | 518 |
| 4 | Lantus Standard | 1566 |
| 8 | Lantus form., AVE0010, Methionin pH 4 | 2053 |
| 25 | Lantus form., AVE0010, Methionin pH 4,5 | 5429 |

weisen Fluoreszenzwerte wie die Referenzformulierungen (Nr. 2 und 4) auf. Bei Werten unterhalb von etwa 6000 liegt keine Fibrillationstendenz vor.

Wenn AVE0010, Lantus und Methionin mit Acetatpuffer mit oder ohne Polysorbat 20 bei pH 4 kombiniert werden, besteht eine höhere Tendenz zur Fibrillation:

| Nr. | Zusammensetzung | Fluoreszenzintensität bei 486 nm |
|---|---|---|
| | | |
| 2 | AVE0010 Standard | 518 |
| 4 | Lantus Standard | 1566 |
| 9 | Lantus form., AVE001 0, ½ Acetat, Met, pH 4 | 18814 |
| 13 | Lantus form., AVE0010, Polysorbat 20, 1/2 Acetat, Met, pH 4 | 22200 |
| 14 | Lantus form., AVE0010, Polysorbat 20, Acetat, Met, pH 4 | 134093 |

Die Werte für die Formulierungen 13 und 14 liegen deutlich über der Schwelle für eine Fibrillationstendenz.

### 6.1 Zusammenfassung

Polysorbat 20 und Polysorbat 80 können zu einer Trübung führen, welche im Doppelbrechungstest nachzuweisen sind. Damit können diese beiden Substanzen zu einer physikalischen Instabilität einer Formulierung von AVE0010 und Insulin führen. Die Zugabe von Methionin führt nicht zu einer physikalischen Instabilität.

### 7. Chemische Stabilität

### 7.1 Stabilität zum Zeitpunkt t0

Die Formulierungen, welche Methionin (mit und ohne Natriumacetat) enthalten, weisen die geringsten Mengen an Verunreinigungen auf (in der Summe etwa 1,2 bis 1,5%). Die folgenden Formulierungen weisen geringe Mengen an Verunreinigungen auf.

| | |
|---|---|
| 8 | Lantus form., AVE0010, Methionin, pH 4 |
| 9 | Lantus form., AVE0010, 1/2 Acetatpuffer, Methionin, pH 4 |
| 11 | Lantus form., AVE0010, Polysorbat 20, Methionin, pH 4 |
| 13 | Lantus form., AVE0010, 1/2 Acetatpuffer, Polysorbat 20, Methionin, pH 4 |
| 14 | Lantus form., AVE0010, Acetatpuffer, Polysorbat 20, Methionin, pH 4 |
| 25 | Lantus form., AVE0010, Methionin, pH 4,5 |
| 26 | Lantus form., AVE0010, 1/2 Acetatpuffer, Methionin, pH 4,5 |
| 28 | Lantus form., AVE0010, Polysorbat 20, Methionin, pH 4,5 |
| 30 | Lantus form., AVE0010, 1/2 Acetatpuffer, Polysorbat 20, Methionin, pH 4,5 |
| 31 | Lantus form., AVE0010, Acetatpuffer, Polysorbat 20, Methionin, pH 4,5 |

Formulierungen, welche kein Methionin enthielten, zeigten einen höheren Anteil an Verunreinigungen.

Polysorbat 20 hat keinen negativen Einfluß auf die chemische Stabilität der Formulierungen.

Acetatpuffer hat dann keinen negativen Einfluß auf die chemische Stabilität, wenn es mit Methionin und Polysorbat 20 kombiniert wird.

Wenn Lysin in den Formulierungen vorhanden ist, ist die Summe der Verunreinigungen größer. Dasselbe gilt für Formulierungen, welche Polysorbat 80, Poloxamer 188 und Benzalkoniumchlorid enthalten.
Die Bestimmung der Verunreinigungen von Insulin Glargin ergab, dass alle Formulierungen vergleichbare Mengen an Verunreinigungen aufwiesen (0,3 bis 0,4%).

### 7.2 Stabilität nach 1 Monat

### 7.2.1 Verunreinigungen von AVE0010

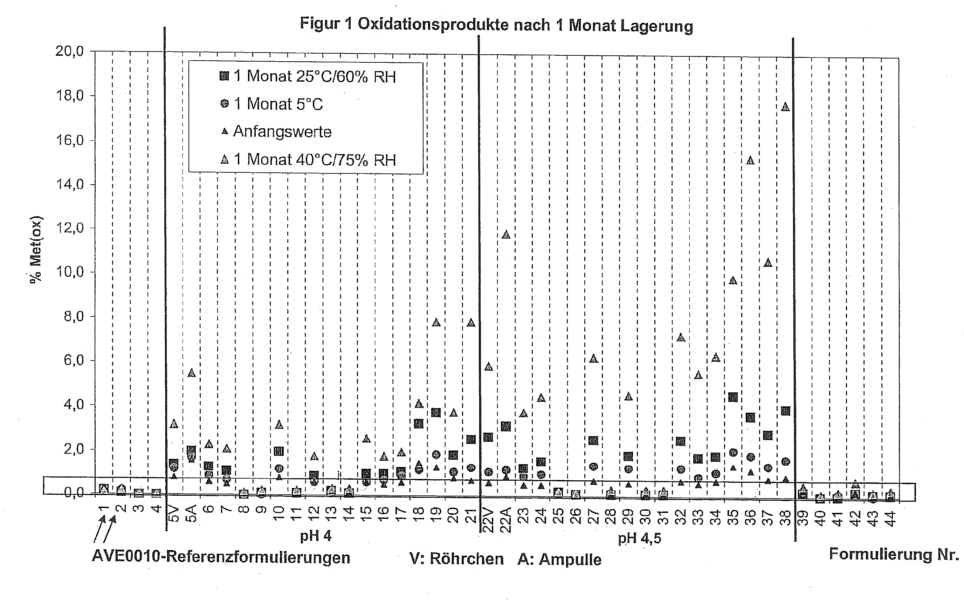

Analysiert wurde der Gehalt an oxidiertem Methionin in den Formulierungen bestimmt. Die Sequenz von AVE0010 weist an Position 14 einen Methioninrest auf. Die Sequenz von Insulin Glargin weist keinen Methioninrest auf. Daher ist der Gehalt an oxidiertem Methionin für eine Oxidation von AVE0010 am Methioninrest indikativ. Die Daten sind in Fig. 1 zusammengefasst. In der Summe zeigen die Daten, dass ohne Methionin bei einem pH-Wert von 4,5 der Anteil an Met(ox) höher ist als bei pH 4,0. Ohne Methionin als Formulierungsbestandteil sind die Anteile von Met(ox) am größten, wenn der Gehalt an Insulin Glargin erhöht ist oder der Gehalt an AVE0010 vermindert ist.

Generell wurden die größten Anteile an Met(ox) bei einer Lagerung bei 40°C/75% rel. Luftfeuchte gemessen. Hierbei finden sich die geringsten Anteile an Met(ox)-AVE0010 (<1%) in den Formulierungen 8, 9, 11, 13, 14, 25, 26, 28, 30 und 31. Die Werte dieser Formulierungen liegen im Bereich der Werte für die AVE0010-Referenzformulierungen Nr. 1 und 2 (Rahmen in Figur 1).

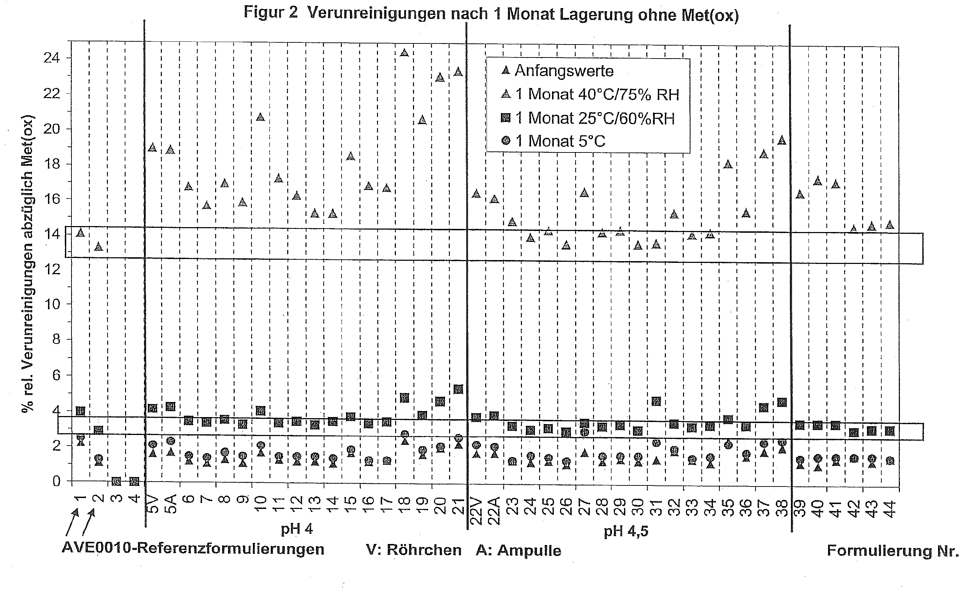

Die Verunreinigungen von AVE0010 nach 1 Monat ohne Met(ox) sind in Figur 2 dargestellt. Die Rahmen zeigen die Werte der AVE0010-Referenzformulierungen bei 25°C bzw. bei 40°C. Formulierungen, die gleiche oder bessere Verunreinigungswerte als die AVE0010-Referenzformulierungen aufweisen, liegen innerhalb der Rahmen oder darunter. Dies trifft auf die Formulierungen 24, 25, 26, 28, 29, 30, 31, 33 und 34 zu (40°C). Verunreinigungswerte, welche oberhalb der Verunreinigungswerte der AVE0010-Referenzformulierungen liegen, deuten auf Verunreinigungen von Insulin Glargin. Generell weisen Formulierungen mit einem pH von 4,5 weniger Verunreinigungen auf als bei einem pH von 4,0.

Die folgenden Formulieren weisen nach Lagerung für einen Monat bei 40°C den geringsten Gehalt an Met(ox) auf und gleichzeitig die geringsten Gehalte an anderen Verunreinigungen (Vergleich der Figuren 1 und 2). Sie sind besser als oder gleich wie die AVE0010-Referenzformulierungen:

| | |
|---|---|
| 25 | Lantus form., AVE0010, Methionin, pH 4,5 |
| 26 | Lantus form., AVE0010, 1/2 Acetatpuffer, Methionin, pH 4,5 |
| 28 | Lantus form., AVE0010, Polysorbat 20, Methionin, pH 4,5 |
| 30 | Lantus form., AVE0010, 1/2 Acetatpuffer, Polysorbat 20, Methionin, pH 4,5 |

Diese Formulierungen gehörten auch zu denjenigen Formulierungen, welche zum Zeitpunkt t0 die geringsten Mengen an AVE0010-Verunreinigungen aufweisen. Alle Formulierungen enthalten Methionin. Polysorbat 20 hat keine negativen Wirkungen auf die Verunreinigungen.

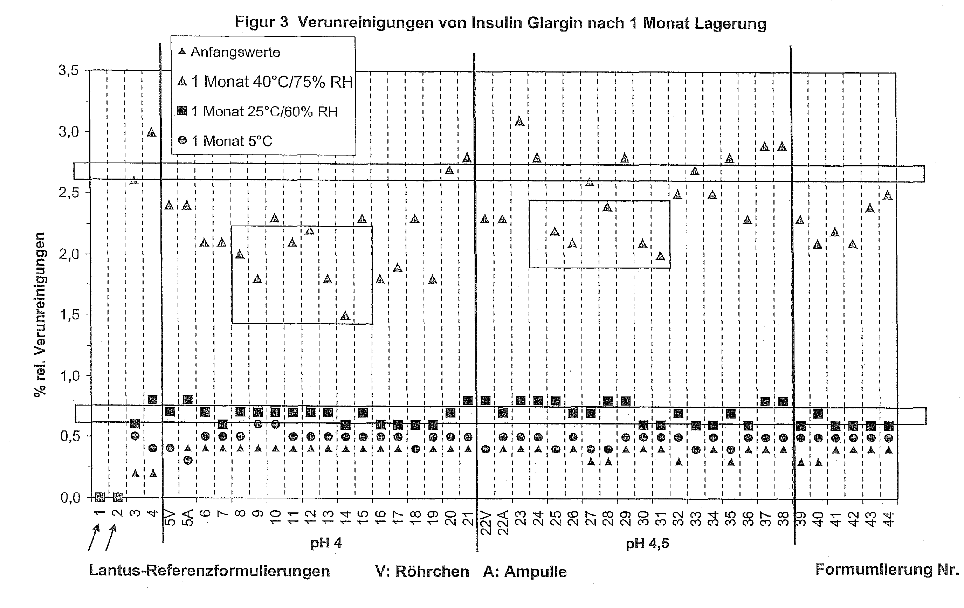

Die Verunreinigungen von Insulin Glargin sind in Figur 3 dargestellt. Die Formulierungen 3 und 4 sind die Referenzformulierungen für Insulin Glargin. Die Werte dieser Formulierungen sind als schmale Rahmen gekennzeichnet. Alle Formulierungen, welche hinsichtlich der AVE0010-Verunreinigungen als beste Formulierungen identifiziert wurden (breite Rahmen, insbesondere die Formulierungen 25, 26, 28 und 30), sind hinsichtlich der Insulin Glargin-Verunreinigungen besser als die Insulin Glargin-Referenzformulierungen (etwa 1,5 bis 2,4 % bei 40°C).

Damit kann aus diesem Experiment geschlossen werden, dass Methionin eine erhöhte Lagerstabilität einer Zusammensetzung umfassend ein Insulin (z.B. Lantus) und einen GLP-1-Agonisten (z.B. AVE0010) bewirkt. Die Zugabe von Methionin bewirkt eine chemische Integrität dieser Zusammensetzung.

### 8. Schlußfolgerungen

Die hierin beschriebenen Daten führen zu folgenden Schlußfolgerungen:
- Methionin führt zu einer erhöhten chemischen Stabilität und hat keine negativen Auswirkungen auf die physikalische Stabilität von Formulierungen einer Kombination von einem GLP-1-Agonisten, insbesondere AVE0010, und einem Insulin, insbesondere Lantus. Daher ist Methionin als Bestandteil dieser Zusammensetzungen vorteilhaft.
- Acetat kann zu einer physikalischen Instabilität führen. Diese Instabilität wird mit steigender Acetatkonzentration größer. Daher sind Formulierungen einer Kombination von einem GLP-1-Agonisten, insbesondere AVE0010, und einem Insulin, insbesondere Lantus, hergestellt, werden, welche frei von Acetat sind, vorteilhaft gegenüber entsprechenden Zusammensetzungen, welche Acetat enthalten.
- Polysorbat 20 hat keinen negativen Einfluss auf die physikalische und die chemische Stabilität von Formulierungen einer Kombination von einem GLP-1-Agonisten, insbesondere AVE0010, und einem Insulin, insbesondere Lantus. Durch Kombination von Acetat in geringeren Konzentrationen (1/2 Acetat) mit Polysorbat 20 können die negativen Wirkungen von Acetat teilweise kompensiert werden. In acetatfreien Zusammensetzungen führt die Zugabe von Polysorbat 20 nicht zu Vorteilen. Daher sollten Formulierungen einer Kombination von einem GLP-1-Agonisten, insbesondere AVE0010, und einem Insulin, insbesondere Lantus, hergestellt, werden, welche frei von Polysorbat 20 sind.
- Lysin (in normalen und höheren Konzentrationen), Benzalkoniumchlorid, Polysorbat 80 und Poloxamer 188 zeigten schon zu Beginn der Untersuchungen (t0) eine chemische Instabilität. Für Lysin trifft dies auch auf die Ergebnisse des THT-Tests zu.

### Beispiel 2

Das "3 pens cover all"-Konzept (Fig. 4)
- 3 Prämix-Stifte (Prämix-Pens) mit 3 verschiedenen vorbestimmten Verhältnissen:
   (e) Mix A: 100 U Lantus + 66.66 µg AVE0010 pro mL
   (f) Mix B: 100 U Lantus + 40 µg AVE0010 pro mL
   (g) Mix C: 100 U Lantus + 25 µg AVE0010 pro mL
- Einsatz der 3 Prämix-Stifte: Die beispielhafte Tabelle in Fig. 4 geht von einem therapeutischen Bereich von 15 bis 80 U pro Dosis Lantus und 10 bis 20 µg AVE0010 aus. Für einen bestimmten Patienten wird eine zu verabreichende Dosis von Lantus festgelegt bzw. vorbestimmt. Die vorbestimmte Dosis wird in der linken Spalte aufgesucht. Sofern in den Spalten MIX A - MIX C eine korrespondierende AVE0010-Dosis im Bereich zwischen 10 und 20 µg genannt ist, wird der entsprechende MIX ausgewählt, dosiert und verabreicht. Die Bereiche sind überlappend: z.B. könnte bei einem Bedarf von 26 bis 30 U Lantus Mix A oder MIX B (mit einer höheren Dosis AVE0010) gewählt werden. Entsprechendes gilt für MIX B und C. Wird zum Beispiel eine Dosis von 50 U Insulin bestimmt, so sind 0,5 ml von MIX B oder MIX C zu dosieren. Diese Dosis enthält 20 µg (MIX B) bzw. 12,5 µg (MIX C) AVE0010.
- Schlußfolgerung: Unter der Annahme, dass eine wahrscheinliche AVE0010-Wirkung zwischen 10 und 15 µg und eine therapeutische Wirkung zwischen 15 und 22 µg erzielt wird, können fast alle Patienten, welche Lantus-Dosen von 15-80 U nehmen, ebenso therapeutische Dosen von AVE0010 zwischen 10 und 20 µg erhalten, wenn sie einen der drei Prämixstifte verwenden, welche drei verschiedene Lantus:AVE0010-Verhältnisse enthalten (Mix A, B oder C). Aufgrund des breiten Bereichs möglicher Verhältnisse von Lantus zu AVE0010 können die Verhältnisse in den Stiften so abgestimmt werden, dass für jede Dosis von Lantus in mindestens einem Stift eine gewünschte Dosis AVE0010 enthalten ist.

## Patentansprüche

1. Flüssige Zusammensetzung umfassend 0,01 bis 1,5 mg/ml, bevorzugt 0,01 bis 0,5 mg/ml, GLP-1-Agonisten oder/und ein pharmakologisch tolerierbares Salz davon, 240 - 3.000 nmol/ml Insulin oder/und ein pharmakologisch tolerierbares Salz davon und gegebenenfalls mindestens einen pharmazeutisch akzeptablen Hilfsstoff, **dadurch gekennzeichnet, dass** sie 0,5-20 mg/ml Methionin enthält.

2. Flüssige Zusammensetzung gemäß Anspruch 1, **dadurch gekennzeichnet, dass** sie einen pharmazeutisch akzeptablen Konservierungsstoff oder/und ein pharmazeutisch akzeptables Isotonisierungsmittel enthält.

3. Flüssige Zusammensetzung gemäß einem der vorhergehenden Ansprüche,
**dadurch gekennzeichnet, dass** sie einen pH-Wert von 3,5 bis 5 aufweist.

4. Flüssige Zusammensetzung gemäß einem der vorhergehenden Ansprüche,
**dadurch gekennzeichnet, dass** der GLP-1-Agonist ausgewählt ist aus der Gruppe bestehend aus GLP-1 und Analoga und Derivaten davon, Exendin-3 und Analoga und Derivaten davon, Exendin-4 und Analoga und Derivaten davon, und wobei der GLP-1-Agonist bevorzugt ausgewählt ist aus der Gruppe bestehend aus desPro36Exendin-4(1-39)-Lys₆-NH₂ und Exendin-4.

5. Flüssige Zusammensetzung gemäß einem der vorhergehenden Ansprüche, wobei das Insulin ausgewählt ist aus Humaninsulinen, Analoga, Derivaten und Metaboliten davon, insbesondere Gly(A21)-Arg(B31)-Arg(B32)-Humaninsulin, LysB28ProB29-Humaninsulin, B28Asp Humaninsulin und B29Lys(ε-tetradecanoyl),desB30 Humaninsulin.

6. Kombination umfassend eine erste pharmazeutische Zusammensetzung gemäß einem der Ansprüche 1 bis 5 und eine zweite pharmazeutische Zusammensetzung gemäß einem der Ansprüche 1 bis 5 und gegebenenfalls wenigstens eine weitere pharmazeutische Zusammensetzung gemäß einem der Ansprüche 1 bis 5, welche jeweils wenigstens ein Insulin und wenigstens einen GLP-1-Agonisten umfassen und das wenigstens eine Insulin oder/und den wenigstens einen GLP-1-Agonisten in unterschiedlichen Gewichtsanteilen bezogen auf das Gesamtgewicht der Zusammensetzung enthalten.

7. Kombination gemäß Anspruch 6, wobei die erste, zweite und die gegebenenfalls weitere(n) Zusammensetzung(en) das wenigstens eine Insulin in einem im wesentlich identischen Gewichtsanteil enthalten und den wenigstens einen GLP-1-Agonisten in unterschiedlichen Gewichtsanteilen enthalten.

8. Kombination gemäß Anspruch 6, wobei die erste, zweite und die gegebenenfalls weitere(n) Zusammensetzung(en) den wenigstens einen GLP-1-Agonisten in einem im wesentlich identischen Gewichtsanteil enthalten und das wenigstens eine Insulin in unterschiedlichen Gewichtsanteilen enthalten.

9. Kit umfassend eine Kombination gemäß einem der Ansprüche 6 bis 8.

10. Zusammensetzung nach einem der Ansprüche 1 bis 5, Kombination gemäß einem der Ansprüche 6 bis 8 oder Kit gemäß Anspruch 9 zur Anwendung in der Behandlung von Diabetes mellitus.

11. Zusammensetzung gemäß einem der vorhergehenden Ansprüche 1 bis 5 zur Verwendung in der Medizin, wobei ferner Metformin, Insulin und/oder ein GLP-1-Agonist und/oder ein pharmakologisch tolerierbares Salz davon gemeinsam verabreicht wird.

12. Zusammensetzung zur Verwendung gemäß Anspruch 11, **dadurch gekennzeichnet, dass** die behandelten Patienten einen HbA1c Wert von 7 % bis 10 % haben.

13. Zusammensetzung zur Verwendung gemäß Anspruch 11 zur Anwendung in der Behandlung von Diabetes Typ II und/oder Obesität.

## Claims

1. Liquid composition comprising 0.01 to 1.5 mg/ml, preferably 0.01 to 0.5 mg/ml, GLP-1 agonists or/and a pharmacologically tolerable salt thereof, 240-3000 nmol/ml insulin or/and a pharmacologically tolerable salt thereof, and, optionally, at least one pharmaceutically acceptable excipient, **characterized in that** it comprises 0.5-20 mg/ml methionine.

2. Liquid composition according to Claim 1, **characterized in that** the composition comprises a pharmaceutically acceptable preservative or/and a pharmaceutically acceptable isotonicity agent.

3. Liquid composition according to either of the preceding claims,
**characterized in that** the composition has a pH in the range from 3.5 to 5.

4. Liquid composition according to any one of the preceding claims,
**characterized in that** the GLP-1 agonist is selected from the group consisting of GLP-1 and analogs and derivatives thereof, exendin-3 and analogs and derivatives thereof, exendin-4 and analogs and derivatives thereof, wherein the GLP-1 agonist is preferably selected from the group consisting of desPro36exendin-4(1-39)-Lys₆-NH₂ and exendin-4.

5. Liquid composition according to any one of the preceding claims, wherein the insulin is selected from the group consisting of human insulins, analogs, derivatives, and metabolites thereof, more particularly Gly(A21)-Arg(B31)-Arg(B32) human insulin, LysB28ProB29 human insulin, B28Asp human insulin, and B29Lys(ε-tetradecanoyl), desB30 human insulin.

6. Combination comprising a first pharmaceutical composition according to any one of Claims 1 to 5 and a second pharmaceutical composition according to any one of Claims 1 to 5, and, optionally, at least one further pharmaceutical composition according to any one of Claims 1 to 5, each comprising at least one insulin and at least one GLP-1 agonist, and containing the at least one insulin or/and the at least one GLP-1 agonist in different weight fractions relative to the total weight of the composition.

7. Combination according to Claim 6, wherein the first, second, and, where used, further composition(s) contain the at least one insulin in a substantially identical weight fraction and the at least one GLP-1 agonist in different weight fractions.

8. Combination according to Claim 6, wherein the first, second, and, where used, further composition(s) contain the at least one GLP-1 agonist in a substantially identical weight fraction and the at least one insulin in different weight fractions.

9. Kit comprising a combination according to any one of Claims 6 to 8.

10. Composition according to any one of Claims 1 to 5, combination according to any one of Claims 6 to 8, or kit according to Claim 9 for application in treating diabetes mellitus.

11. Composition according to any one of preceding Claims 1 to 5 for use in medicine, metformin, insulin, and/or a GLP-1 agonist, and/or a pharmacologically tolerable salt thereof additionally being administered together.

12. Composition for use according to Claim 11, **characterized in that** the treated patients have an HbA1c value in the range from 7% to 10%.

13. Composition for use according to Claim 11 for application in treating type II diabetes and/or obesity.

## Revendications

1. Composition liquide comprenant 0,01 à 1,5 mg/ml, de préférence 0,01 à 0,5 mg/ml d'agonistes du GLP-1 et/ou un sel pharmacologiquement tolérable de celui-ci, 240-3.000 nmol/ml d'insuline et/ou un sel pharmacologiquement tolérable de celle-ci et éventuellement au moins un adjuvant pharmaceutiquement acceptable, **caractérisée en ce qu'**elle contient 0,5-20 mg/ml de la méthionine.

2. Composition liquide selon la revendication 1, **caractérisée en ce qu'**elle contient un conservateur pharmaceutiquement acceptable et/ou un agent d'isotonisation pharmaceutiquement acceptable.

3. Composition liquide selon l'une quelconque des revendications précédentes, **caractérisée en ce qu'**elle présente un pH de 3,5 à 5.

4. Composition liquide selon l'une quelconque des revendications précédentes, **caractérisée en ce que** l'agoniste du GLP-1 est choisi dans le groupe constitué par le GLP-1 et des analogues et dérivés de celui-ci, l'exendine-3 et des analogues et dérivés de celle-ci, l'exendine-4 et des analogues et dérivés de celle-ci, et l'agoniste du GLP-1 étant de préférence choisi dans le groupe constitué par le desPro³⁶Exendine-4(1-39)-Lys₆-NH₂ et l'exendine-4.

5. Composition liquide selon l'une quelconque des revendications précédentes, l'insuline étant choisie parmi les insulines humaines, les analogues, les dérivés et les métabolites de celles-ci, en particulier la Gly(A21)-Arg(B31)-Arg(B32)-insuline humaine, la Lys^{B28} Pro^{B29}-insuline humaine, la B28Asp-insuline humaine et la B29Lys(ε-tétradécanoyl),desB30-insuline humaine.

6. Combinaison comprenant une première composition pharmaceutique selon l'une quelconque des revendications 1 à 5 et une deuxième composition pharmaceutique selon l'une quelconque des revendications 1 à 5 et éventuellement au moins une autre composition pharmaceutique selon l'une quelconque des revendications 1 à 5, qui comprennent à chaque fois au moins une insuline et au moins un agoniste du GLP-1 et qui contiennent ladite au moins une insuline et/ou ledit au moins un agoniste du GLP-1 en des proportions pondérales différentes par rapport au poids total de la composition.

7. Combinaison selon la revendication 6, la première, la deuxième et la/les autre(s) composition(s) éventuelle(s) contenant ladite au moins une insuline en une proportion pondérale sensiblement identique et ledit au moins un agoniste du GLP-1 en des proportions pondérales différentes.

8. Combinaison selon la revendication 6, la première, la deuxième et la/les autre(s) composition(s) éventuelle(s) contenant ledit au moins un agoniste du GLP-1 en une proportion pondérale sensiblement identique et ladite au moins une insuline en des proportions pondérales différentes.

9. Kit comprenant une combinaison selon l'une quelconque des revendications 6 à 8.

10. Composition selon l'une quelconque des revendications 1 à 5, combinaison selon l'une quelconque des revendications 6 à 8 ou kit selon la revendication 9 pour l'application dans le traitement du diabète sucré.

11. Composition selon l'une quelconque des revendications précédentes 1 à 5 pour l'utilisation dans la médecine, en outre la metformine, l'insuline et/ou un agoniste du GLP-1 et/ou un sel pharmacologiquement tolérable de ceux-ci étant conjointement administrés.

12. Composition pour l'utilisation selon la revendication 11, **caractérisée en ce que** les patients traités présentent une valeur HbA1c de 7 à 10%.

13. Composition pour l'utilisation selon la revendication 11 pour l'application dans le traitement du diabète de type II et/ou de l'obésité.
